# EUROPEAN PATENT SPECIFICATION

(11) **EP 2 212 344 B1**
(45) Date of publication and mention of the grant of the patent: **26.03.2014**
(21) Application number: 08802131.6
(22) Date of filing: 12.09.2008
(51) Int. Cl.: C07K 14/475, A61K 9/00, A61K 38/00, A61P 19/02

(54) **USE OF SLIT, NEPHRIN, EPHRIN OR SEMAPHORIN FOR TREATMENT OF CARTILAGE DISEASES**
VERWENDUNG VON SLIT, NEPHRIN, EPHRIN ODER SEMAPHORIN ZUR BEHANDLUNG VON KNORPELERKRANKUNGEN
UTILISATION DE POLYPEPTIDES SLIT, NEPHRIN, EPHTRIN OU SEMAPHORINE POUR LE TRAITEMENT DE MALADIES DU CARTILAGE

(30) Priority: 14.09.2007 EP 07018131; 03.04.2008 EP 08006810
(43) Date of publication of application: 04.08.2010
(73) Proprietor: Formycon AG, 82152 Planegg (DE)
(72) Inventor: BOSSERHOFF, Anja, 93053 Regensburg (DE); SCHUBERT, Thomas, 60318 Frankfurt (DE); HUSTERT, Elisabeth, 82110 Germering (DE)
(74) Representative: Dey, Michael
(86) International application number: PCT/EP2008/007579
(87) International publication number: WO 2009/033715

(56) References cited:
- WO-A-00/55321
- WO-A-02/06315
- WO-A-02/081745
- WO-A2-2005/074556
- WO-A2-2008/073441
- US-A1- 2003 032 057
- US-A1- 2005 014 680
- DATABASE WPI Week 1999 Thomson Scientific, London, GB; AN 1999-411830 XP002480435 "New vertebrate Slit protein useful for diagnosis and treatment of cancers in nerves, muscle and endocrine system." & JP 11 164690 A (ASAHI KASEI KOGYO KK) 22 June 1999 (1999-06-22)
- KLAGSBRUN M ET AL: "A role for axon guidance receptors and ligands in blood vessel development and tumor angiogenesis" CYTOKINE AND GROWTH FACTOR REVIEWS, OXFORD, GB, vol. 16, no. 4-5, 1 August 2005 (2005-08-01), pages 535-548, XP004991797 ISSN: 1359-6101
- 19000101, 1 January 1900 (1900-01-01), XP009100317

## Description

The present invention is related to the use of a repellent factor and/or polypeptide, in particular of a SLIT3 protein or fragments thereof for manufacturing of a pharmaceutical composition for treating or preventing degenerative diseases in particular cartilage defects. The invention is further related to a polypeptide fragment of SLIT3, a pharmaceutical composition comprising the polypeptide and the use thereof.

Cartilage repair and regeneration in traumatic and degenerative joint diseases remains still a major challenge in orthopaedics. Cartilage injuries and degenerative disorders play a major reason which creates significant pain and gradual loss of joint function with disability and immobility in the adult population. The intrinsic repair capabilities of cartilage are limited owing to the low mitotic potential of chondrocytes *in vivo* and the resulting tissue produced in adults is generally inferior to normal cartilage. It is generally believed that damaged cartilage does not receive sufficient stimuli to elicit a repair response because cartilage e.g. articular cartilage lacks a vasculature. Chondrocytes in cartilaginous tissue are normally not exposed to sufficient amount of repair stimulating agents such as growth factors and fibrin clots present in damaged vascularised tissue.

The functional integrity of joints depends on strict separation of the compartment borders. Crossing of the joint compartment boundaries and destruction of the cartilage compartment is found in inflammatory and degenerative joint diseases such as rheumatoid arthritis (RA) and osteoarthritis (OA).

Frequently cartilage disorders (e.g. rheumatoid arthritis) are characterized or aggravated by the invasive and destructive behaviour of cell populations from surrounding tissue compartments such as synovial tissue fibroblast (SF) cells migrating in from synovial tissue. The destruction of articular structures of the joint, like cartilage and bone, through synovial fibroblasts (SF) is a crucial event in RA and OA (Pap, 2003; Pap, 2007; Yasuda, 2006; Huber, et al., 2006).

Numerous strategies to inhibit further progression of the destructive structural changes as well as novel approaches to reverse the disease by regenerative treatment of diseased cartilage including finding of new agents that have the potential to repair destructed cartilage have been performed with varying degree of success. These strategies include marrow-stimulation techniques, autologous and allogenic transplantation, autologous chondrocyte transplantation, periosteal grafting, pridie drilling, abrasion chondroplasty, including microfracturing and mosaicplasty. However, these procedures are not reliable and are limited to small- and medium- sized focal chondral and osteochondral defects. Mosaicplasty is limited by the need to create defects at donor sites, by an insufficient repair result and by technical difficulties experienced in resurfacing the original curvature of the joint (Kuroda, et al., 2007). The autologous chondrocyte transplantation techniques has several disadvantages which includes lack of sufficient number of chondrocytes, necessity of creation of donor site defects, poor efficacy and high treatment costs.

Several growth factors like TGF-ß1, FGF-2, IGF-1, TP-508, OP-1 or other BMPs including GDFs or combinations thereof have been identified that induce the proliferation and differentiation of progenitor cells into functional bone and/or cartilage tissue. In particular, TGF-ß1 has been shown to promote chondrogenic differentiation of progenitor cells and to enhance chondrocyte differentiation. IGF-1 primarily acts in an anabolic fashion to increase proteoglycan and type II collagen synthesis. However, these growth factors are rather unspecific having the risk for undesired side effects. Others such as BMPs have the potential to induce osteogenic genes and therefore induce undesired bone formation. Another limitation of the application of this growth factor technology is the relatively short biological half-life of the exogenous growth factor that would enable only transient biological effects after their delivery.

Pro-inflammatory cytokines, proteases and metalloproteinases are likely responsible for some signs and symptoms as well as structural changes present in OA and RA patients. The inhibition of the synthesis/activity of these enzymes as a treatment for OA and RA have been the focus of intensive research.

To date, the most promising strategy is still the use of small chemical entities that can block the activity of MMPs. However, MMP inhibitors could produce side effects and have not yet demonstrated a major reduction in the progression of the disease.

In addition, other new strategies are currently under development which are cited below.

US2003068705 discloses three hundred and five nucleic acids encoding PRO polypeptides of which some (e.g. IL-17 homologous or related polypeptides) are allegedly useful for stimulating the proliferation or differentiation of chondrocytes and as such may be used in the treatment of various bone and/or cartilage disorders such as sport injuries or arthritis.

WO20001090357 provides proteins for the treatment of developmental defects, inflammatory disease, or for modulating immune responses.

US2003044917 relates to human PRO polypeptides for stimulating the proliferation and differentiation of chondrocytes and their use in the treatment of various bone and/or cartilage diseases such as arthritis and sports injuries. However, only sequence 6029 was tested positive.

Another class of proteins such as axonal guidance molecules (e.g. Netrins, Slits) has been suggested for modulating inflammatory cell movement (WO0164837, WO05074556, WO00055321, W00190357). WO0164837, WO03075860, WO06019904 and US 2006/0153840 disclose methods for promoting angiogenesis, proliferation and/or promoting migration of cells for treatment of neuropathy and cancer.

WO2002081745 teaches the use of SLIT3 for diagnosis of osteoporosis/bone disease, promoting osteogenesis and/or preventing osteoporosis/bone disease.

WO99/23219, JP11164690 and JP11075846 disclose the human SLIT3 polypeptide sequence.

WO00/55321 discloses vertebrate SLIT DNA sequences (i.e. Xenopus and human Slit2), protein and uses thereof.

However, none of the methods and compositions disclosed in the art demonstrates a major reduction in the progression of the treated disease without side effects or results in abolishment of destructive processes and actual repair and replacement of cartilage tissue.

Therefore, there is a need for compositions and uses thereof for an improved treatment and/or prevention of progressive cartilage destruction, in particular, after trauma and in case of chronic diseases such as full-thickness defects and superficial defects and arthritis (e.g. osteoarthritis, rheumatoid arthritis), meniscal tears, anterior crucial ligament (ACL)-injury, which overcome the problems associated with the currently available methods and compositions.

Therefore, an object of the present invention is to provide means for repairing or regeneration of cartilage defects and/or inhibition of the disease progression of cartilage diseases or disorders such as arthritis and sport injuries.

Another object of the present invention is to provide means for inhibition of synovial cell migration and therefore inhibition of cartilage destruction.

Another object of the present invention is to provide means, which are suitable in the treatment of cartilage diseases and more particularly inflammatory cartilage diseases and diseases with involvement of synovial fibroblast invasion or pannus formation.

Another object of the present invention is to provide a composition and use thereof for attraction/chemotaxis of mesenchymal stem cells from the surrounding for cartilage regeneration.

Another object of the present invention is to provide compositions and uses thereof for the induction of cartilage formation and/or stimulating the proliferation or differentiation of chondrocytes.

The problem underlying the present invention is solved by the use of a repellent factor for the manufacture of a pharmaceutical composition for the prevention and/or treatment of a cartilage defect, wherein the repellent factor is selected from Slits.

The invention, in particular, relates to the use of a repellent factor for manufacturing of a pharmaceutical composition for the prevention and/or treatment of a degenerative disease, preferably a degenerative cartilage disease a) associated with infiltration or migration of synovial fibroblasts preferably into articular or non-articular cartilage and/or associated with pannus formation, or b) associated with decreased attachment of cells preferably fibroblast or synovial fibroblast cells to articular or non-articular cartilage. In particular, the repellent factor modulates or inhibits the migration of fibroblast-like cells such as synovial fibroblast cells, inhibition of synovial cell hyperplasia and/or promotes migration, proliferation and/or differentiation of mesenchymal stem cells and/or chondrocytes for cartilage induction and/or regeneration and thereby alters, inhibits, prevents and/or treats the progression of the degenerative disease. In particular, the repellent factor inhibits, alters or prevents osteophyte formation, preferably in a patient.

In particular, the invention relates to the use of a repellent factor for the manufacture of a pharmaceutical composition for the prevention and/or treatment of a cartilage defect, wherein the repellent factor is a fragment of SLIT3 (SEQ ID NO:1). The fragment preferably has a length of ≤ 1523 AA, preferably ≤ 1200 AA, more preferably ≤ 1000 AA and most preferably ≤ 800 AA and, in particular, ≥ 20 AA, preferably ≥ 50 AA, more preferably ≥ 100 AA. Preferably, the fragment of SLIT3 comprises the sequence of SEQ ID NO:1 from position 34 to 917, position 47 to 863, position 66 to 857, position 279 to 863, position 279 to 857, position 279 to 724, position 279 to 504, position 311 to 432, position 335 to 432 or position 312 to 437. According to the invention it has been found that repellent factors, in particular, SLIT3 are expressed at the cartilage compartment borders where they maintain the integrity of the articular cartilage. Further repellent factors inhibit migration of fibroblasts, nerve fibers and vessels into articular cartilage. Since invasion of pannus tissue into cartilage in chronic joint disease is only possible after altered repellent factor expression, this invasion can be modulated by repellent factors, in particular, by SLIT3 and, consequently, degenerative diseases associated therewith can be treated.

The inventors have demonstrated that members of the repellent factors and their receptors are differentially expressed in synovial fibroblasts of patient suffering from degenerative diseases such as osteoarthritis and arthritis. This was analyzed by gene quantitative RT-PCR performed on a Lightcycler of cDNA isolated from cultured primary human synovial fibroblasts. ROBOs and SLITs are similarly expressed in synovial fibroblasts of osteoarthritis (OASF) patients and synovial fibroblasts of rheumatoid arthritis (RASF) patients except of ROBO3 mRNA expression which was strikingly enhanced in RASF compared to a very weak expression in OASF. SLIT1 mRNA levels are clearly lowered compared to SLIT2 or SLIT3, but expression of SLITs in OASF appears to be generally higher than in RASF.

ROBO1 and ROBO2 are expressed both in differentiated and OA chondrocytes, but expression in OA chondrocytes is 10-fold (ROBO1) and even 100-fold (ROBO2) increased compared to differentiated chondrocytes. No expression of ROBO3 was measured in both cell types. All three SLITs are expressed in differentiated and OA chondrocytes, finding again higher levels in OA than in differentiated chondrocytes.

UNC5B and UNC5C were significantly upregulated in synovial fibroblasts of OA and RA patients.

Further, it has been found that the significant loss of ROBO3 expression in RASF in higher passages of synovial fibroblast cells might be an important step in loosing repulsive activity towards SLIT3. These findings presented by the inventors suggest that deregulation of the ROBO3 receptor in synovial fibroblasts in RA (and OA) correlates with aggressiveness of the fibroblasts.

Furthermore, the invention is based on the finding that repellent factors such as SLIT3 as well as fragments thereof are involved in regulation of the aggressive behaviour of cells such as synovial fibroblasts cells and migration of these cells to or into cartilage, which is a major problem in degenerative diseases such as rheumatoid arthritis. Without being limited to specific molecules, the invention is based on the finding that repellent factors such as SLIT3 and fragments thereof inhibit the migration of OASF and RASF cells. Due to this surprising finding, repellent factors can be used for the treatment of a variety of diseases which are subject to the migration and infiltration of cells including altered migration of fibroblast-like cells such as synovial fibroblast cells and/or pannus formation which can't subsequently cause destruction or altered function of a tissue such as articular or non-articular cartilage. In view of the surprising finding disclosed herein, repellent factors can be used as a medicament for the prevention and/or treatment of various diseases and disease conditions described herein. Respective diseases are, among others, degenerative preferably cartilage diseases such as degenerative joint and/or disc diseases including those further described above. In particular, according to the invention, the disease is selected from degenerative disc disease, meniscus tears, anterior crucial ligament (ACL) injury, arthritis, osteoarthrithis, rheumatoid arthritis, psoriatic arthritis, juvenile chronic arthritis, rhizomelic pseudoarthritis, rheumatoid polyarthritis, synovitis or villonodular synovitis.

According to the invention cartilage defects or cartilage diseases can be prevented or treated.

Another finding of the inventors was the influence of repellent factors on the migratory behavior of human mesenchymal stem cells which are known for their ability to differentiate into chondrocytes. Induction of migration was observed using a Boyden Chamber assay after adding factors such as NETRIN1 to the lower chamber, indicating that repellent factors can act as a chemoattractant for mesenchymal stem cells such as synovial membrane or lining cells. In view of this finding, not only the destructive behavior of migratory cells can be inhibited but also cells for regeneration of destructed tissue can be attracted. Repellent factors therefore further improve the treatment of degenerative diseases.

A further finding was the induced differentiation of human mesenchymal stem cells towards a chondrogenic cell lineage showing that repellent factors such as SLIT3 can induce chondrogensis and therefore are useful for regeneration and treatment of cartilage diseases.

Therefore, the invention encompasses the use of slit repellent factors for manufacturing a pharmaceutical composition for treatment of a cartilage defect, in particular, a degenerative disease in a patient caused by aggressive fibroblast cells by administration of an effective amount of a repellent factor that modulates the migration, proliferation and/or attachment of cells to cartilage.

Preferably, an effective amount of a repellent factor administered to a patient is from 0.001 mg/kg body weight of patient to 100 mg/kg body weight of patient, preferably 0,01 mg/kg body weight of patient to 20 mg/kg body weight of patient, more preferably from 0.01 to 10 mg/kg body weight of patient, most preferably 0,1 mg/kg to 1 mg/kg body weight of patient.

The term "repellent factor" means an active agent, which at a certain concentration or concentration range is able to inhibit the migration of cells across a tissue boundary e.g. the cartilage boundary. As such for example the migration of aggressive synovial fibroblasts into the cartilage and/or bone can be inhibited or altered. It also includes inhibition or further progression of pannus formation or pannus invasion into cartilage. Repellent factors can alter the sprouting of nerve fibers as axon guiding cues for example by repellent or attractant activities.

Repellent factors comprise a large number of molecules also known as axon guidance molecules which modulate axonal outgrowth in the nervous system. They include NETRINs, SLITs, SEMAPHORINS and EPHRINS and its receptors NEUROPILIN, ROUNDABOUTs (ROBOs), and members of the UNC5 and DCC families (Klagsbrun and Eichmann, 2005).

NETRINS are a family of secreted proteins with homology to laminin domains. This family consists of NETRIN1, 2, 3 und 4. They contain a laminin-VI domain, EGF-like repeats and a heparin-binding C-terminal domain. Netrin receptors are the deleted in colorectal cancer (DCC) receptor family, which consists of DCC and neogenin, or the uncoordinated-5 (UNC5) family, which comprises UNC5A, 5B, 5C, 5D. Netrins have been implicated in angiogenesis, and during morphogenesis such as lung branching, mammary gland development and tumorgenesis. NETRIN binding to DCC and neogenin was shown to lead to cell attraction whereas binding to the UNC receptor modulates repulsion.

SLITs were originally identified in *Drosophila* as axon guidance molecules. Mammalian SLIT1, SLIT2 and SLIT3 orthologs are large secreted proteins with four leucine-rich repeat (LRR) domains, nine EGF domains, a laminin G (LamG) domain, and C-terminal cystine knot. SLIT1 mRNA is expressed in brain, anaplastic oligodendroglioma, and Jurkat T cells. SLIT2 and SLIT3 mRNAs are co-expressed in embryonic stem (ES) cells with embryoid body formation (Katoh and Katoh, 2005; Vargesson, et al., 2001). Slit3, but not Slit2, is localized within the mitochondria which implies that Slit3 has potentially unique functions not shared by other Slit proteins (Little, et al., 2001). Slits act by binding to Roundabout receptors. There are four human Robo transmembrane receptors (Robo1, Robo2, Robo3 and Robo4) that share fibronectin type III and immunoglobuline-like domains (Ig), but vary in their cytoplasmatic domains (Itoh, et al., 1998; Howitt, et al., 2004; Hohenester, et al., 2006). Slit proteins are primarily known to be involved in regulating cell-cell interactions and cell-matrix interactions of migrating cells during embryonic development and by mediating axon guidance through attraction or repulsion of growth cones. Over the last decade, the Robo-/ Slit system has also been described to mediate cell adhesion and induce tumor angiogenesis. Slits are also implicated in other developmental processes such as muscle precursor cell migration in drosophila, leucocyte trafficking and kidney induction (Klagsbrun and Eichmann, 2005).

Semaphorins are surface or locally secreted nerve repellent factors with a specific repellent action on nerve fibers via distinct surface receptors such as neuropilin 1 and neuropilin 2. Class III Semaphorins include SEMA A, B, C, and F. Ephrins and their receptors Eph represent ligands and tyrosine kinase receptors implicated in a variety of developmental processes including the nervous system development and angiogenesis. Ephrin A ligands (Ephrin A1, A2, A3, A4, A5) are GPI anchored molecules which bind to EphA receptors, whereas Ephrin B proteins (Ephrin B1-3) bind to Eph B receptors. Ephrins can also vice versa function as receptors which are bound by Ephs as ligands.

The repellent factor may be an agonist or antagonist of a ROUNDABOUT receptor (e.g. ROBO1, 2, 3), Deleted in Colon Cancer (e.g. DCC) or UNC (e.g. UNC5A-D) receptor. As used herein, the agonist or antagonist activates or inhibits the activity of a ROUNDABOUT receptor, DCC or UNC receptor, which preferably is the signal transduced by the interaction between the receptor and the ligand or the inhibition of a signal that is transduced by the receptor which is not occupied by the ligand.

According to the present invention, the repellent factor is selected from the group of SLITs (such as SLIT1, SLIT2, SLIT3), fragments thereof and amino acid sequences thereof.

The term "treating or treatment" as used herein, means an alleviation of a disorder or disease or an alleviation of symptoms associated with a disorder or disease, halt of further progression or worsening of the symptoms. The term "prevention" includes prevention or prophylaxis of the disease or disorder.

The term "cartilage defect" refers to any cartilage abnormality including cartilage diseases, alteration of cartilage caused e.g. by trauma or degenerative processes.

The term "degenerative diseases" means diseases or defects with impaired cartilage structure such as cartilage degeneration or destruction with or without involvement of bony structures. Preferably, degenerative diseases are degenerative cartilage diseases. These include temporomandibular joint disorder (TMDs), acetabular labrum disorders, arthritis, osteoarthritis, rheumatoid arthritis, psoriatic arthritis, juvenile chronic arthritis, rhizomelic pseudoarthritis, rheumatoid polyarthritis, degenerative disc disease, osteochondral defects, superficial chondral defects, osteochondritis dissecans, full-thickness chondral defects, partial-thickness chondral defects, meniscus tears, anterior crucial ligament injury, synovial osteochondromatosis, ankylosing spondylitis, synovitis, villonodulat synovitis.

The terms cartilage defect and degenerative diseases as used herein, in particular, do not include osteoporosis.

The term "degenerative disc disease (DDD)" is a chronic process characterized in part by progressive loss of proteoglycan and water content in the nucleus pulposus of one or more discs that can become manifest in multiple disorders such as idiopathic low back pain, disc herniation, internal disc disruption or fissured discs, radiculopathy, spinal stenosis, herniated nucleus pulposus-induced sciatica, sciatica, idiopathic scoliosis and/or myelopathy. The disc degeneration grade can be ranked by analysis of preoperative MRI.

The term "articular cartilage" covers the surface of the portion of bones in joints and functions as a cushion between two bones to allow movement in joints. Normal healthy cartilage is described as hyaline cartilage. Articular cartilage consists of specialized cells (chondrocytes) embedded into a matrix of intracellular material rich in proteoglycans, predominantly aggrecan, collagen type II fibrils, other proteins and water. The matrix is produced and maintained by the chondrocytes embedded within. Cartilage tissue is not innervated and vascularised and is nourished by the underlying tissue.

Non-articular cartilage does not cover articulating surfaces and includes fibrocartilage (including interarticular fibrocartilage e.g. the meniscus and fibrocartilage of the intervertebral disc) and elastic cartilage.

The term "synovial cells", "synovium" or "synovial fibroblasts" as used herein, refers to a cell physiologically associated with the synovial membrane or present in the synovial space; a cell obtained from the joint's synovial membrane or synovial fluid. It also refers to cellular lining covering the non-cartilaginous surfaces of the synovial joints. The synovium consist of the synovial lining cell and the synovial subling (subsynovium), which merges with the joint capsule.

The term "pannus tissue" means a proliferation of synovial cells and/or an erosion of tissue such as a deposit of cells or material onto cartilage (e.g. articular cartilage), an invasion or destruction of articular cartilage with or without involvement of the subchondral region. Also included is an invasion of cells into the subchondral bone marrow.

In a preferred embodiment the problem underlying the present invention is solved by the use of a repellent factor for manufacturing of a pharmaceutical composition for the prevention and/or treatment of a degenerative disease which preferably is not osteoporosis, wherein the repellent factor is selected from the group consisting of polypeptides having a sequence which a) has at least 85% homology, preferably 90%, 95% homology, more preferably identity to the amino acid sequence of SLIT3 (SEQ ID NO: 1), b) is an active fragment of SLIT3 or c) has at least 85%, 90%, 95% homology to the amino acid sequence of the active fragment of b).

The term "active fragment" preferably means an activity of the polypeptide to bind to the same receptor or binding partner as the mature full-length polypeptide e.g. the mature SLIT3 polypeptide. Binding for example may activate or inhibit the receptor and downstream cascade. SLIT3 fragment activity can for example be determined by binding to its ROBO receptor using methods know in the art such as a Biacore binding assay, an in vitro pulldown assay as described in (Marlot et al., 2007), ELISA protein binding assay or immunoprecipitation. Activity of the fragment can also be determined using an assay as described within Examples 2, 3, 4, 6, 7 or 9 of the invention e.g. Boyden Chamber migration assay, proliferation assay or an *in vivo* functional assay such as described in Examples 6 and 7.

To determine the percent homology of two amino acid sequences, the sequences are aligned for optimal comparison purposes (e.g., gaps can be introduced in the sequence of one protein or nucleic acid for optimal alignment with the other protein or nucleic acid). The amino acid residues at corresponding amino acid positions are then compared. When a position in one sequence is occupied by the same amino acid residue as the corresponding position in the other sequence, then the molecules are homologous at that position.

The percent homology between the two sequences is a function of the number of identical positions shared by the sequences (i.e., % homology = numbers of identical positions/total numbers of positions x 100).

In addition to the above-described methods, a determination of the percent homology between two sequences can be accomplished using a mathematical algorithm. A preferred, non-limiting example of a mathematical algorithm utilized for the comparison of two sequences is the algorithm of Karlin and Altschul (1990 Proc. Natl. Acad. Sci. USA 90:5873-5877). Such an algorithm is incorporated into the NBLAST and XBLAST programs of Altschul, et al. (1990 J. Mol. Biol. 215:403-410).

BLAST protein searches can be performed with the XBLAST program, score=50, wordlength=3 to obtain amino acid sequences homologous to CCSRPs of the present invention. To obtain gapped alignments for comparison purposes, Gapped BLAST can be utilized as described in Altschul et al. (1997 Nucleic Acids Res. 25:3389-3402). When utilizing BLAST and Gapped BLAST programs, the default parameters of the respective programs (e.g., XBLAST and NBLAST) can be used. Another preferred non-limiting example of a mathematical algorithm utilized for the comparison of sequences is the algorithm of Myers and Miller (CABIOS 1989). Such an algorithm is incorporated into the ALIGN program (version 2.0) that is part of the GCG sequence alignment software package. When utilizing the ALIGN program for comparing amino acid sequences, a PAM120 weight residue table, a gap length penalty of 12 and a gap penalty of 4 can be used to obtain amino acid sequences homologous to the CCSRPs of the present invention. To obtain gapped alignments for comparison purposes, Gapped BLAST can be utilized as described in Altschul et al. (1997 Nucleic Acids Res. 25:3389-3402). When utilizing BLAST and Gapped BLAST programs, the default parameters of the respective programs (e.g., XBLAST and NBLAST) can be used. Another preferred non-limiting example of a mathematical algorithm utilized for the comparison of sequences is the algorithm of Myers and Miller (CABIOS 1989). Such an algorithm is incorporated into the ALIGN program (version 2.0) that is part of the GCG sequence alignment software package. When utilizing the ALIGN program for comparing amino acid sequences, a PAM120 weight residue table, a gap length penalty of 12 and a gap penalty of 4 can be used.

Fragments preferably have a length of less than 1523 or 1522 amino acids, preferably of less than 1491, 900, 500, 400, 300, 200 amino acids, preferably at least 20, 50, 100 amino acids and up to 100, 200, 300, 400, 500 amino acids. In a preferred embodiment the repellent factor according to the invention comprises the amino acid sequence selected from SEQ ID NO: 2, 3, 4, 5, 6, 7, 8 and 9 and preferably a polypeptide, the amino acid sequence of which is shorter than the mature SLIT3 protein. Preferably, the amino acid sequence is shorter than 1523 or 1522, preferably shorter than 1491 or shorter than 900 amino acids in length, preferably between 200 and 900 amino acids in length or more preferably between 220 and 760, between 240 and 520, between 250 and 300 amino acids in length. In a further preferred embodiment the repellent factor according to the invention comprises at least the amino acid sequence of SEQ ID NO: 6, 7, 8 or 9 and the amino acid sequence is shorter than the mature SLIT3 protein.

The term "mature SLIT3 protein" refers to the human SLIT3 protein without a signal peptide sequence. Preferably, the mature SLIT3 protein starts at position 33 or 34 of SEQ ID NO: 1 or starts at position 1 or 2 of SEQ ID NO: 2.

In some embodiments, the repellent factor comprises the amino acid sequence selected from SEQ ID NO: 1 to 9 or has at least 85% homology to the amino acid sequence of SEQ ID NO:1 to 9, wherein the amino acid sequence preferably is shorter than the mature SLIT3 protein.

In another preferred embodiment, the repellent factor according to the invention comprises at least the amino acid sequence of SEQ ID NO:1 from position 34 to 917, position 47 to 863, position 66 to 857, position 279 to 863, position 279 to 857, position 279 to 724, position 279 to 504, position 311 to 432, position 335 to 432 or position 312 to 437. More preferably, the repellent factor according to the invention comprises at least the amino acid sequence of SEQ ID NO:1 from position 308 to 413, position 308 to 317, position 331 to 340, position 344 to 352, position 357 to 366, position 380 to 389, position 394 to 402 or position 404 to 413 or a combination thereof.

Further preferred are the repellent factors, in which one or more Cys are substituted by another amino acid, in particular, by Ala. Preferred are repellent factors comprising a sequence selected from SEQ ID NO:1 to 9, in particular, from SEQ ID NO:2 to 9, in which one or more Cys are substituted by another amino acid, in particular, by Ala. A preferred Example of such repellent factor is SEQ ID NO:21 which is derived from SEQ ID NO:9 by substituting Cys in position 85 with Ala.

In a preferred embodiment, the repellent factor is non-glycosylated at least in the second leucine-rich repeat domain or homologous leucine-rich repeat domain.

The term "second leucine-rich repeat domain" of the present invention refers to a conserved leucine-rich amino acid sequence present in SLIT proteins. The leucine-rich repeat (LRR) domain of SLIT3 consist of four LRRs (LRR1-4) flanked by amino- and carboxy-terminal conserved regions (LRR-NR and LRR-CR) (Itoh, et al., 1998; Howitt, et al., 2004; Hohenester, et al., 2006). The second leucine rich domain encompasses the amino acid sequence between position 248 and 467 of SEQ ID 1. Homologous leucine-rich repeat domain refer to an amino acid sequence with conserved amino acid exchanges of the second leucine-rich repeat domain, the homologous region of other SLIT family members such as SLIT1 and SLIT2 and/or a homology of 80%, 85%, 90%, 95% to the amino acid sequence between position 248 and 467 of SEQ ID1.

In a further preferred embodiment, the repellent factor according to the present invention is non-glycosylated, more preferably the repellent factor is a recombinant *E.coli* derived protein.

In a further preferred embodiment, the degenerative disease preferably degenerative cartilage disease is selected from the group consisting of degenerative disc disease, meniscus tears, anterior crucial ligament (ACL) injury, arthritis, osteoarthritis, rheumatoid arthritis, psoriatic arthritis, juvenile chronic arthritis, rhizomelic pseudoarthritis, rheumatoid polyarthritis, villonodular synovitis or synovitis.

In another preferred embodiment, the articular cartilage is hyaline articular cartilage.

In a preferred embodiment, the non-articular cartilage is selected from the group of meniscus and intervertebral disc.

In some embodiment, the pharmaceutical composition of the present invention and/or the pharmaceutical composition comprising the isolated polypeptide of any of the embodiments of the invention is an injectable formulation which preferably is injected locally near the defect or non-systemically or even more preferably administered intra-articularly, into the synovia and/or into the synovial fluid.

In a further preferred embodiment, the pharmaceutical composition of the present invention and/or the pharmaceutical composition comprising the isolated polypeptide of any of the embodiments of the invention is an injectable formulation administered systemically e.g. by intravenous administration.

In a specific embodiment, the pharmaceutical composition is administered locally to the area in need of treatment. This may be achieved by for example local infusion during surgery, topical application, by injection, by means of a catheter or by means of an implant, said implant being porous or non-porous.

Other routes for administration of pharmaceutical compositions covered by the present invention are intradermal, intramuscular, intraperitoneal, subcutaneous, intranasal or oral routes. The dosage range adopted will depend, among others, on the route of administration and on age, weight and condition of the patient being treated.

A suitable amount of polypeptide administered to a patient is preferably from 0.001 mg/kg body weight of patient to 100 mg/kg body weight of patient, preferably 0,01 mg/kg body weight of patient to 20 mg/kg body weight of patient, more preferably from 0.01 to 10 mg/kg body weight of patient, most preferably 0,1 mg/kg to 1 mg/kg body weight of patient.

The pharmaceutical composition comprising the repellent factor of the present invention and/or the pharmaceutical composition comprising the isolated polypeptide of any of the embodiments below and above may be in a variety of formulations e.g. pharmaceutical acceptable carriers. Examples of suitable pharmaceutical carriers are described in "Remington's Pharmaceutical Sciences, 18th Ed., 1990, ISBN #: 0912734027, Mack Pub Co). The formulation may deliver the repellent factor rapidly or may be a sustained release formulation. Such compositions may contain a therapeutically effective amount of the repellent factor together with a suitable amount of carrier so as to provide the form for proper administration to the patient. The formulation should suit the mode of administration. Regardless of the route of administration, the repellent factor is formulated into acceptable unit dosage forms by conventional methods known to the person skilled in pharmaceutical art. In a preferred embodiment, the repellent factor is formulated in accordance with routine procedures as a pharmaceutical composition for intravenous administration to human beings. Typically, the pharmaceutical compositions for intravenous administration include a solubilization agent e.g. sterile isotonic aqueous buffer with or without a local anesthetic such as lignocaine. Generally, the ingredients are supplied either separately or mixed together in unit dosage forms for example as a dried lyophilized powder or water-free concentrate in a hermetically sealed container such as an ampoule or sachet. In case where the pharmaceutical composition is to be administered by infusion, it can be dispersed with an infusion bottle containing a solubilization agent.

In a preferred embodiment the formulation includes aqueous liquid, saline solution, solid, semi-solid, liquid, pasty, putty dosage forms, dispersions, suspensions, gels, liposomes, a matrix, a shape stable material, *in situ* setting materials, a tissue adhesive biomaterial, injectable and minimal invasive as well as implantable application form.

In some other embodiment, the pharmaceutical composition of the present invention is selected from the group consisting of an aqueous liquid, a solid, semi-solid, paste, putty, liposomes, a shape stable material, a matrix, an *in situ* setting material, a tissue adhesive biomaterial.

In a preferred embodiment, the pharmaceutical composition comprises the repellent factor and/or the isolated polypeptide of the repellent factor encapsulated in liposomes. The liposomal formulations have the advantage over solutions in that the protein is protected against degradation within the biological environment in addition to a longer duration of the therapeutic agent and slower clearance at the site of application.

Liposomes generally comprise an enclosed lipid droplet having a core typically containing a compound in an aqueous medium. In certain embodiments, the compound is chemically bound to a lipid component or simply contained within the aqueous inside compartment of the liposome.

Pharmaceutical compositions provided according to the present invention are preferably provided as dried liposomal compositions that can be reconstituted to produce liposomes encapsulating the repellent factor and/or the polypeptide, preferably the liposomal preparation are dried reconstituted vesicles (DRVs) which upon reconstitution in an aqueous solution form protein encapsulated liposomes. The liposomal composition used herein is for example dry granular products which upon addition of water disperse to form multi-lamellar liposomal formulations comprising the biological active component. Advantageously, stability problems such as aggregation or oxidation are avoided by using dried liposomes.

Suitable lipids for use in the formulations which are present individually or in mixtures include neutral or positively charged lipids such as cholesterol, phosphatidylcholine, hydrogenated phosphatidylcholine, distearoyl-phosphatidylcholine, sphingomyelin, dioleyl phosphatidylcholine, dioleylphosphatidylglycerol, phosphatidylglycerol, dimyristoylphosphatidyl-choline, dipamlitoylcholine, gangliosides, ceramides, phosphatidyinositol, phosphatic acids, dicetylphosphate, dimyrylstoyl phosphatidylcholine, stearylamine, dipalmitoyl phosphatidylgycerol and other similar lipids. Preferably the lipid mix is charged. The liposomal formulation is typically a mixture of at least two lipids such as cholesterol and phosphatidylcholine and more usually three or more lipids.

The present invention further provides an isolated polypeptide having at least 85% amino acid sequence homology, preferably 90%, more preferably 95% homology to the amino acid sequence of SEQ ID NO: 1 to 9 and wherein the amino acid sequence is shorter than the mature SLIT3 protein, preferably wherein the isolated polypeptide is shorter than 1523, preferably shorter than 1491 or shorter than 900 amino acids in length, preferably between 200 and 900 amino acids in length or more preferably between 200 and 760, between 200 and 520, between 200 and 300 amino acids in length. In a preferred embodiment, the isolated polypeptide comprises the amino acid sequence selected from the group consisting of SEQ ID NO: 1 to 9, more preferably the isolated polypeptide contains the amino acid of SEQ ID NO: 1, 2, 3, 4, 5, 6, 7, 8 or 9 and wherein the amino acid sequence is shorter than the mature SLIT3 protein, preferably wherein the isolated polypeptide is shorter than 1523, preferably shorter than 1491 or shorter than 900 amino acids in length, preferably between 200 and 900 amino acids in length or more preferably between 220 and 760, between 240 and 520, between 250 and 300 amino acids in length.

In a preferred embodiment, the present invention further provides an isolated polypeptide which comprises at least the amino acid sequence of SEQ ID NO:1 from position 34 to 917, position 47 to 863, position 66 to 857, position 279 to 863, position 279 to 857, position 279 to 724, position 279 to 504, position 311 to 432, position 335 to 432 or position 312 to 437.

More preferably, the present invention provides an isolated polypeptide which comprises at least the amino acid sequence of SEQ ID NO:1 from position 308 to 413, position 308 to 317, position 331 to 340, position 344 to 352, position 357 to 366, position 380 to 389, position 394 to 402 or position 404 to 413 or a combination thereof.

In a preferred embodiment, the isolated polypeptide and/or the repellent factor of the present invention is pegylated. The thus modified polypeptide and/or the repellent factor has a biological half-life time greater than the unmodified agent and therefore improves efficacy of the agent for medical treatment of degenerative disorders. In addition tendency towards aggregation of the protein is reduced.

Pegylation can be achieved via stable covalent bonds between an amino or sulfhydryl group on the protein and a chemically reactive group (carbonate, ester, aldehyde, or tresylate) on the polyethylenglycol (PEG). The resulting structure may be linear or branched. PEG reagents are for example described in Roberts et al. (Roberts, et al., 2002). Other pegylation agents are but are not limited to methoxypoly(ethylene glycol) (mPEG), methyl PEO₁₂ maleimide PEG, amine-reactive, methyl-capped polyethylene oxide (PEO)-containing modification agents (Methyl PEOₙ-NHS Esters, n=4, 8,12).

Also modified and/or stabilized peptides can be employed such as stabilized peptides having modified bondings to prevent degradation of the peptides.

The present invention further provides a pharmaceutical composition comprising an isolated polypeptide or repellent factor of any of the above embodiments.

In another preferred embodiment, the invention provides a pharmaceutical composition of any of the embodiments wherein the protein is combined with a carrier, implant or delivery system.

The carrier, implant or delivery device used in the invention is preferably biocompatible in that it is not toxic and does not elicit inflammatory reactions in the body. They can include a matrix or scaffold structure and may be a solid, a liquid, a gel, a paste or other injectable form. Preferably, the carrier, implant or delivery device comprise a hydrogel as for example described in WO2005/113032, in particular injectable hydrogels, sulphated hyaluronic acid, a highly substituted carboxymethylcellulose and salts thereof, alginate, hydroxypropylalginate, chitosan, hydroxethylstarch, collagen, gelatin, reverse thermal gels (e.g. Pluronic F128), a chitosan based thermosensitive copolymer (e.g. chitosan-Pluronic® hydrogel), a porous silk scaffold, a plurality of microspheres, a liposomal formulation and a hydroxyapatite fiber mesh.

In a preferred embodiment, the carrier according to the present invention comprises a fibrin gel composed of platelet-rich plasma, platelet enriched plasma with biodegradable gelatin hydrogel, fibrin/hyaluronic acid composites, factor encapsulated gelatine hydrogel microspheres, injectable biodegradable hydrogel composites comprising for example polymers such as oligo(poly(ethyleneglycol) fumarate, polylactide (PLA) /polyglycolicacid (PGA) and poly epsilon capronolactone.

Preferably, the carrier comprises a chitosan-glycerol phosphate or an *in situ* blood clot stabilized with chitosan-glycerol phosphate solution.

In another embodiment, the delivery system comprises a degradable hydrogel scaffold such as gelatin microparticles, polymer based biodegradable and biocompatible hydrogels, a polymer-reinforced hybrid clot or other polymer based biodegradable and biocompatible implants or delivery systems for delivery of the repellent factor and/or isolated polypeptide of the present invention.

Preferred polymers of the invention comprise collagen, chondroitin sulfate, hyaluronic acid, hyaluronate, hyaluronic esther, hyaluronic benzylester, oligo(poly)(ethylene glycol) fumarate, chitosan, chitosan glycerol phosphate, including tri-copolymers such as gelatin/chondroitin-6-sulfate/hyaluoran tri-copolymer or any combination thereof.

In a further embodiment, the pharmaceutical composition is formulated as a sustained release formulation. In another embodiment, the pharmaceutical composition is formulated as a sustained release formulation which provides intermittent release.

Treating the degenerative disease can be further improved by stabilizing a blood clot formed in the lesion of the degenerated tissue with a polymer that has thrombogenic activity and stimulates the wound repair process. Therefore, in a further preferred embodiment, the pharmaceutical composition further comprises at least one substance, additive or matrix with thrombogenic activity.

In a further preferred embodiment, the polymer is thrombogenic and/or stimulates platelet aggregation and degranulation.

In another preferred embodiment, the polymer is hemostatic, more preferably, the polymer is hemostatic and stimulates revascularization of the wound tissue and/or connective tissue repair.

In another embodiment, the polymer is solubilized under physiological conditions and forms a solid implant after mixture with whole blood. These implants have the advantage to adhere more to the tissue defect than normal clots and therefore improve cartilage regeneration.

In some embodiment, the pharmaceutical composition of any of the embodiments of the present invention further comprises cartilage cells, preferably wherein the cartilage cells are chondrocytes.

Co-encapsulation of human chondrocytes derived from various tissues e.g. human ear, nasal, rib, inner meniscus cells (IMCs), fat pat cells (FPCs), synovial membrane cells (SMCs), articular chondrocytes or other chondrocyte tissue, mesenchymal stem cells (MSCs) and/or bone marrow stroma cells, in either of the above mentioned embodiments or a combination thereof is also an embodiment of the invention In particular, these cells are expanded with or without growth factors to enhance chondrogenesis of different chondrocyte types and then induce to form cartilage tissue after implantation.

Mesenchymal stem cells (MSCs)" according to the present invention are a primitive or resting cell population that resides in many mature skeletal tissues as uncommitted mesenchymal progenitor cells. MSCs are flexible and have the ability to differentiate towards several mature tissue types, including cartilage, bone, fat and other tissue, depending on the environment and biological signals provided to these resting cells. MSCs are available from many autologous sources, including bone marrow, blood, muscle tissue and fat that can be harvested to isolate these cells without significant donor site morbidity or immunogenic potential.

Furthermore, the problem underlying the present invention is solved by the use of a polypeptide of any of the above embodiments for manufacturing of a pharmaceutical composition for the prevention and/or treatment of a disease such as a degenerative disease. In a preferred embodiment, the degenerative disease is a degenerative disease as specified in of any of the above embodiments. Preferably the degenerative disease is arthritis such as osteoarthritis or rheumatoid arthritis including chronic or acute rheumatoid arthritis.

Further herein described is the use of a repellent factor according to any of the above mentioned embodiments for influencing the migratory behavior of mesenchymal stem cells.

Further herein described is the use of a repellent factor according to any of the above mentioned embodiments for induction of migration or attraction of mesenchymal stem cells.
Preferably, the invention relates to the use of a repellent factor according to any of the above mentioned embodiments for the induction of cartilage formation and/or for stimulation of the proliferation or differentiation of chondrocytes.

Further herein described is an isolated polypeptide comprising the consensus sequence C(Y)₃CYC(Y)₆C(Y)₉₉C(Y)₄₇CYC(Y)₂₀C(Y)₂₀C(Y)₂₀C(Y)₁₃₂₂C.

Further described is an isolated polypeptide comprising the consensus sequence C(Y)₃CYC(Y)₆C(Y)₉₉C(Y)₄₇CYC(Y)₂₀C(Y)₂₀C(Y)₂₀C(Y)₁₃₋₂₂C, wherein C denotes cysteine and Y denotes any amino acid, wherein at least one cysteine is substituted by any other amino acid except cysteine. By replacing at least one cysteine by another amino acid folding and/or dimer formation of the sequence can be influenced.

Further herein described is a pharmaceutical composition for gene therapy for the prevention and/or treatment of a degenerative disease comprising a DNA of a repellent factor gene or parts thereof, which encodes for a repellent factor or active fragment thereof which has a therapeutic effect on a degenerative disease such as one disease described within the invention.

In the pharmaceutical composition, the DNA of a repellent factor gene or parts thereof can be provided preferably with a retroviral vector, adenoviral vector, adenovirus-associated viral (AAV) vector, herpes simplex virus (HSV) vector, murine leukemia virus (MLV) vector, lentivirus vector or a plasmid which can be expressed in a human cell or a recombinant cell which has been transformed or transduced with a recombinant vector comprising the DNA encoding for the repellent factor or active fragment thereof in order to apply the repellent factor to the impaired tissue locally (local administration), systemically or continuously.

The term "DNA repellent factor gene" means a DNA encoding for a repellent factor or active fragment thereof. It is not limited to the natural isolated DNA but also includes any form of modification of the natural nucleotide sequence which after translation forms a polypeptide of any of the embodiments of the present invention or which forms a polypeptide under maintenance of the activity of the repellent factor preferably in addition to elements for expression regulation operably linked to the nucleic acids (e.g. inducible promoter elements and/or enhancer element(s)) suitable for the purpose of the present invention. The recombinant vector also may comprise a nucleotide sequence for a signal peptide required for secretion of the polypeptide by the gene out of the cell. The vector may further comprise one or more selection marker gene or reporter gene.

The DNA may be derived from a human, mouse, rat or other vertebrate repellent factor. Nucleotide sequences encoding these proteins are readily available to a skilled person. Preferably, the DNA is derived from a human repellent factor. The sequences can be for example found in GenBank, SwissProt and other databases. They may be cDNA or genomic DNA and clones comprising the nucleic sequences can be derived from depositories such as the ATCC or can be cloned by PCR approach or can be chemically synthesized. The DNA sequence may be further optimized for codon usage. Examples of DNA sequences for repellent factors are GI4507060 (Slit1), GI4759145 (Slit2), GI11321570 (Slit3), GI:148613883 (Netrin1) or nucleic acid fragments thereof.

Preferably, the recombinant cells are autologous fibroblasts of the synovium, skin, bone marrow origin, Imyphocytes or dendritic cells. Most preferably, the recombinant cells are autologous fibroblasts of the synovium, skin or dendritic cells.

It is one embodiment of the present invention to provide a pharmaceutical composition and use thereof for locally delivering nucleic acid molecules encoding for the repellent factor or an active fragment thereof to an affected tissue such as arthritic joints in particular to the rheumatoid synovium. In particular, the pharmaceutical composition and use thereof enables an efficient transfection or transduction of nucleic acid molecules encoding a repellent factor in cells of tissue such as synovial cells in a therapeutically effective amount and over a sufficient period of time with preferably high expression of the therapeutic protein in the target cells.

Preferably, the vector or plasmid is not integrated into the genome of a transduced cell e.g. to express the protein in an extrachromosomal manner.

Preferably, the vector or plasmid is stably integrated into the genome of a transduced cell and preferably provides long term and/or sustained release of the therapeutic protein.

Local administration of the pharmaceutical composition to the affected tissue such as arthritic joint refers preferably to *in vivo* or *ex vivo* administration to the arthritic synovium. Delivery of the nucleic acid ex vivo comprises transduction of autologous or non-autologous synovial cells with the viral vector or transfection with a plasmid comprising the nucleic acid encoding the repellent factor or parts thereof, selecting the transduced cells and administering the transduced cells to the rheumatoid joint of the subject by for example reimplantation, injection or reinfusion of cells into the joint of the subject. *In vivo* administration refers to the direct administration of the vector or plasmid comprising the nucleic acid encoding the repellent factor or parts thereof to the joint of the subject e.g. by intra-articular injection. The advantage of local administration is that less gene product and thus fewer vector is needed to treat individual joints.

For systemical delivery the vector is preferably introduced at extra-articular locations by for example intramuscular, subcutaneous or intravenous injection by single or repeated injection or infusion.

Preferably, the pharmaceutical composition of the above embodiments further comprises additional pharmaceutical acceptable excipients such as but not limited to physiological liquids, water, saline, buffers, emulsifiers, stabilizers, lyophilisation agents and the like.

As an alternative the pharmaceutical composition for gene therapy for the prevention/and or treatment of a degenerative disease comprising a DNA of a repellent factor gene or parts thereof, which encodes for a repellent factor or active fragment thereof is introduced into liposomes and is injected directly into the area of the joint, where the liposomes fuse with the synovial cells resulting in an *in vivo* expression of the repellent factor of any of the embodiments above.

Preferably, the pharmaceutical composition of any of the above embodiments is injected as naked DNA.

The invention is further illustrated by the Figures and Examples which are merely illustrative and are not constructed as a limitation of the scope of the present invention.
**FIGURE 1** shows the inhibitory effect of NETRIN1 on the migration of synovial fibroblasts. The effect of NETRIN1 on OA (A) and RA (B) synovial fibroblasts in passage 3 and passage 10 was measured in Boyden Chamber assays. NETRIN1 was applied to the upper or the lower chamber, respectively.
**FIGURE 2** shows the migration of OASF and RASF after addition of Slit3 analyzed in a Boyden Chamber Migration Assay.
   Slit3 incubation (0.1 µg/ml) decreased migration of OASF in early passages (P3 and P4) to about 60% (Slit3 in upper chamber together with OASF) compared to control and about 40% (Slit3 in lower chamber). In RASF (P3/P4), Slit3 reduced migration to about 45% (Slit 3 in upper chamber) and 50% (Slit3 in lower chamber) respectively.

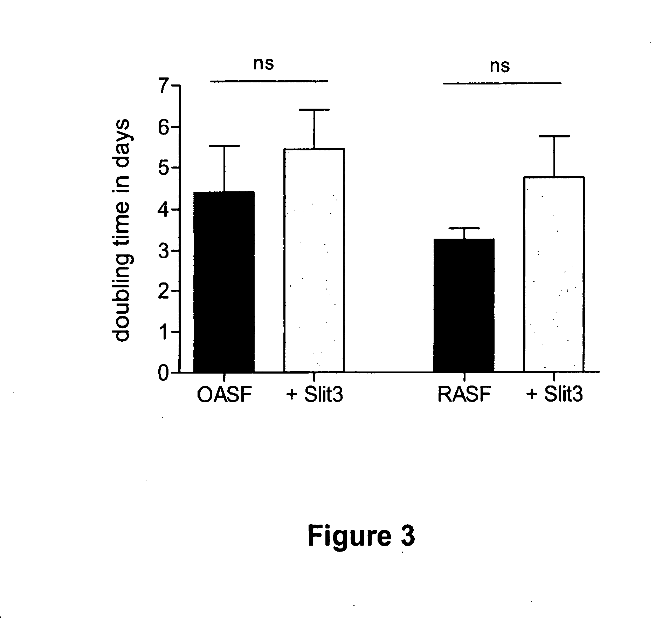

**FIGURE 3** shows the influence of Slit3 on the proliferation of synovial fibroblasts of osteoarthritis (OASF) patients and rheumatoid arthritis (RASF) patients. Slit3 incubation (0,1 µg/ml) together with OASF and RASF decreased growth of OASF and RASF slightly.

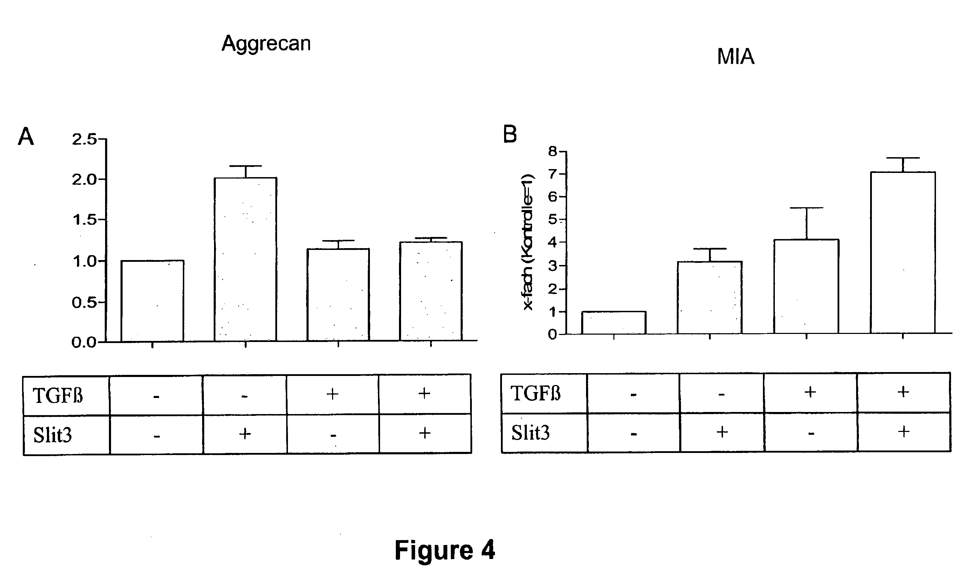

**FIGURE 4** shows the differentiation of Human Mesenchymal Stem Cells (HMSC) upon Slit3 addition.
   **A** Slit3 increased aggrecan expression after 7 days of treatment.
   **B** TGFß induced differentiation of HMSCs towards chondrocytes was further enhanced by Slit3 treatment analyzing MIA expression.

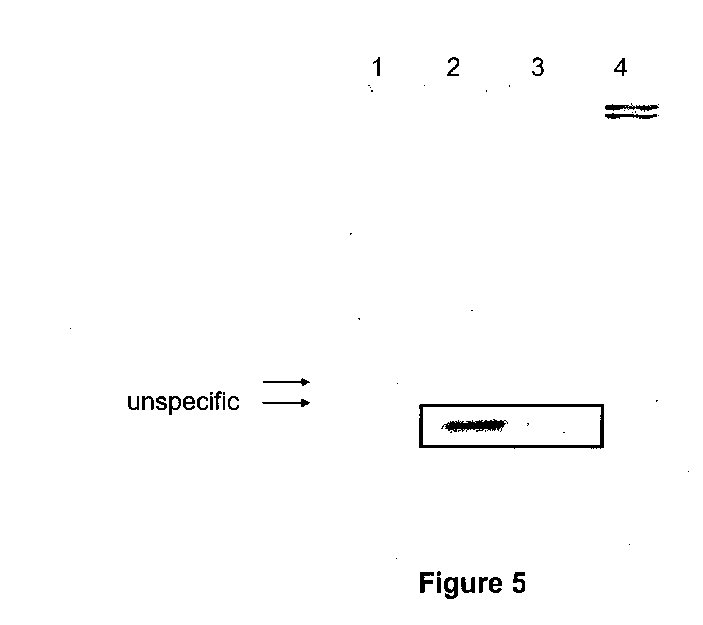

**FIGURE 5** shows the expression of the LRR2 construct expressed with the RTS 500 system (Roche Molecular Biochemicals) on SDS PAGE.
   Lane 1: recombinant protein of RTS 500 (without His-Tag) as control 10 µl
   Lane 2: recombinant SLIT3 fragment LRR2 10µl
   Lane 3: recombinant SLIT3 fragment LRR2 1µl
   Lane 4: recombinant mouse Slit3 (250ng; R&D Systems)

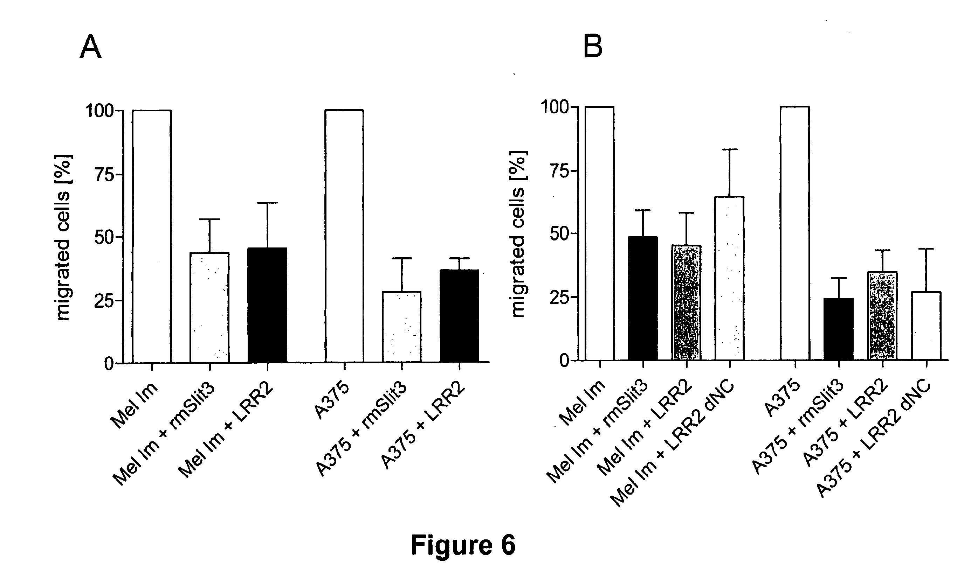

**FIGURE 6** shows the SLIT3 mediated inhibition on the migration of Mel Im (R&D Systems) and A 375 cells using Slit3 (R&D Systems), LRR2, LRR2 dNC.

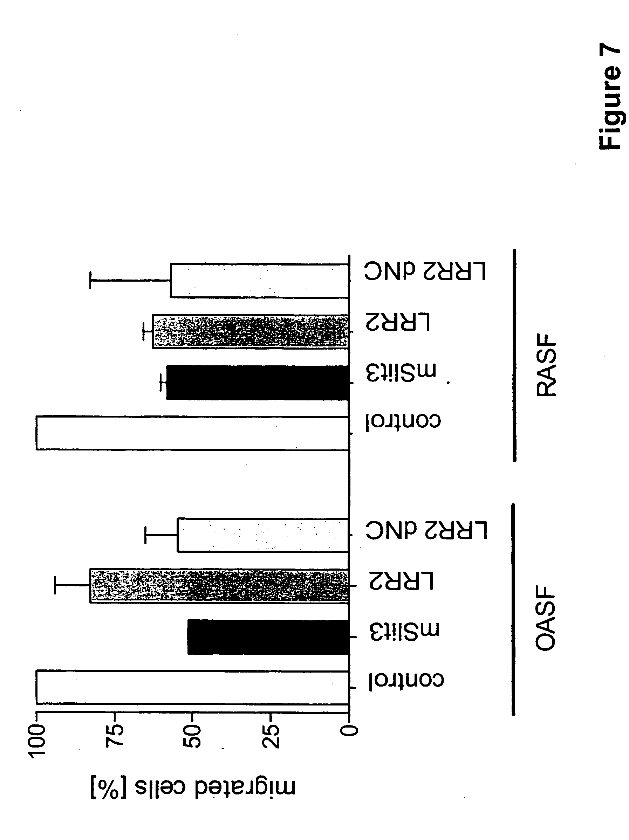

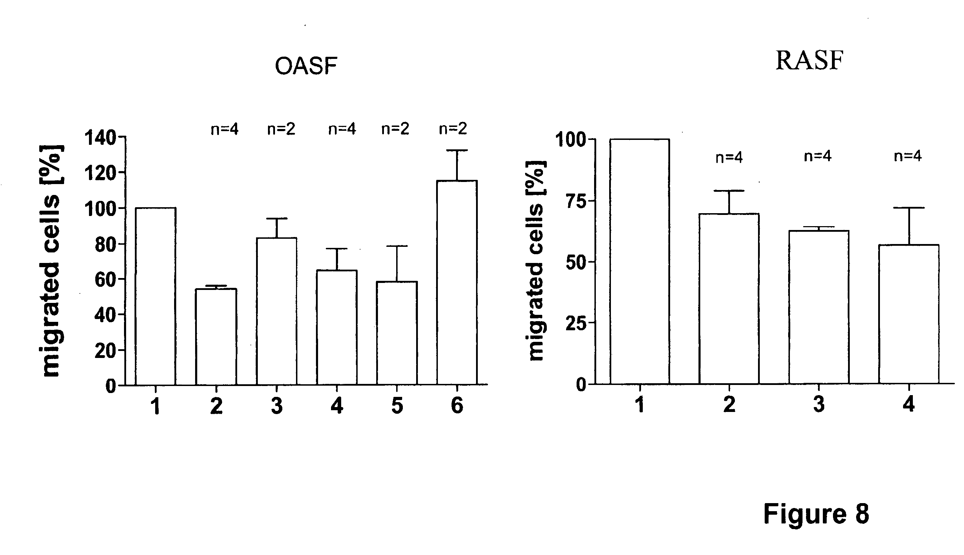

**FIGURE 7** **and** **8** show reduced migration of OASF and RASF in a Boyden Chamber Migration Assay (according to example 2 and 5) in early passages upon treatment with Slit 3 (R&D Systems, Fig. 7, Fig. 8 lane 2, LRR2 (Fig. 7, Fig. 8 lane 3), LRR2 dNC (Fig. 7, Fig. 8 lane 4), LRR 2dNCmut (Fig. 8 lane 5) and LRR3 (Fig. 8 lane 6). Lane 1: control

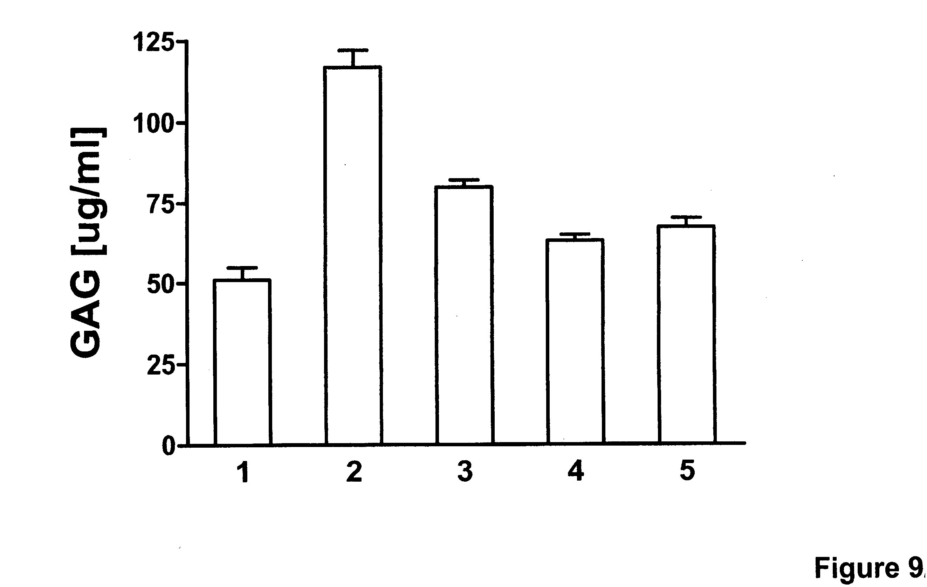

**FIGURE 9** shows the inhibition of cartilage destruction measured as GAG protein level in the supernatant of OA synovial fibroblasts after co-culture with different repellent proteins.
   Lane 1: cartilage control (without OA synovial fibroblasts)
   Lane 2: OA synovial fibroblasts
   Lane 3: OA synovial fibroblasts + NETRIN1
   Lane 4: OA synovial fibroblasts + mSlit3
   Lane 5: OA synovial fibroblasts + LRR2 dNCmut

### Example 1: Isolation of primary human synovial fibroblasts

Synovial tissue samples were obtained from synovectomy and arthroplastic surgery from four patients with RA and four patients with OA (Clinic of Orthopedic Surgery, Schulthess Clinics, Zuerich, Switzerland) after informed consent and approval of the local ethics committee. The diagnosis of OA was made according to clinical and radiological criteria. All RA patients fulfilled the American College of Rheumatology 1987 criteria for the diagnosis of RA. In OA patients, synovial tissue was obtained from hip and knee joints exhibiting end-stage joint space narrowing, whereas in RA patients, material was sampled from wrist or proximal interphalangeal joints with the joints exhibiting florid synovitis and/or arthritic destructions. Synovial tissue was minced mechanically, washed extensively in sterile phosphate-buffered saline (PBS) and digested with 150 mg/ml Dispase II (Boehringer Mannheim, Mannheim, Germany) for 1h at 37 C under continuous agitation. The resulting cell suspension was seeded into tissue culture dishes and cultured in Dulbecco's modified Eagle's medium (DMEM; Gibco Life Technologies, Basel, Switzerland) containing 10% fetal calf serum and 100 U/ml penicillin per 100 µg/ml streptomycin in a humidified atmosphere at 37 C followed by the addition of 5% CO₂. In the third, fifth and tenth passage, adhering fibroblasts were washed, trypsinized and used for RNA isolation or in the assays. As a control dermal fibroblasts were used.

### Example 2: Inhibition of migration of primary human synovial fibroblasts from RA and OA patients

The inhibitory potential of repellent factors on the migration of synovial fibroblasts from osteoarthritis and rheumatoid arthritis patients was analyzed in a migration assay.

The effect of the repellent factors on cell migration was analyzed using a Boyden Chamber assay containing polycarbonate filters with 8 µm pore size (Costar, Bodenheim, Germany), essentially as described previously (Jacob, et al., 1995). Filters were coated with gelatin. The lower compartment was filled with fibroblast-conditioned medium, used as a chemo-attractant. Cells were harvested by trypsinization for 2 min, resuspended in DMEM without FCS at a density of 2 x 10⁵ cells/ml and placed in the upper compartment of the chamber. After incubation at 37°C for 4 hours, the filters were collected and the cells adhering to the lower surface fixed, stained and counted. The repellent factor e.g. recombinant NETRIN1 (human recombinant NETRIN1 protein derived from HEK293 cells, amino acid 28-604, N-terminal Flag-tag, Alexis Biochemicals), Slit3 (recombinant mouse Slit3 protein, Ser27 - His 901, R&D Systems) was added either to the upper or the lower chamber of the system in a concentration of 0.1 µg/ml.

It is know that phenotypical changes in synovial fibroblasts occur during cultivation *in vitro* and that these cells lose their aggressive phenotype gradually while grown *in vitro* (Zimmermann et al., 2001). Therefore, the inventors desired to obtain freshly isolated synovial fibroblast cells with phenotypic features as close as possible to the configuration observed *in vivo* and analyzed the effect of repellent factors on the migration using cells in early and late passages.

These experiments clearly demonstrated the inhibition of these factors on the migration of freshly isolated synovial fibroblasts from human synovial tissue of patients with RA and OA.

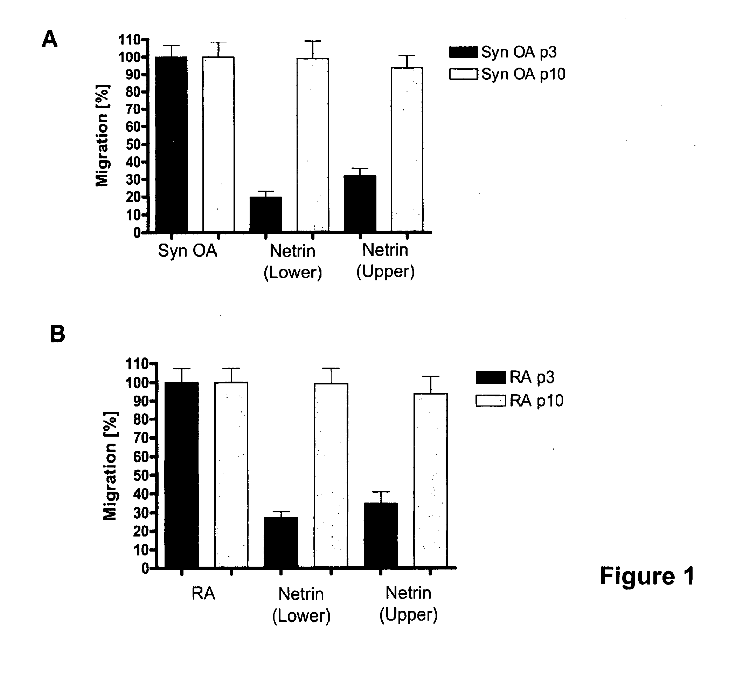

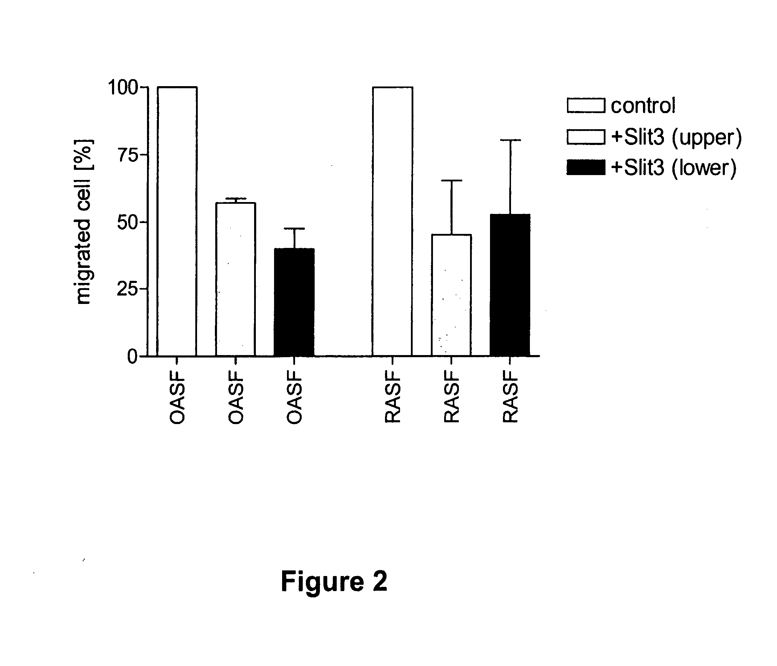

The results are depicted in Figs. 1 and 2.

As depicted in Fig. 1 (A) and Fig. 1 (B), there was a strong inhibition of RA and OA synovial fibroblast migration in early passages (passage 3) whereas fibroblasts derived from healthy donors or in high passages were not affected. The effects were seen after addition of recombinant NETRIN1 to the upper and to the lower chamber, respectively, indicating that not chemotaxis but molecular processes of migration were affected by NETRIN1. These findings presented here suggest that NETRIN reduces the migratory ability of the cells, potentially by repulsion of the cells.

As depicted in Fig. 2, migration of OASF and RASF and the influence of the guidance cue Slit3 on the cells were analyzed. In early passages (P3 and P4), a strong inhibitory effect of Slit3 on both OASF and RASF (n=2) compared to untreated cells was determined. This effect was evident not only in presence of Slit3 in the upper chamber together with the SF, but also in combination of Slit3 with the fibroblast-conditioned medium as chemoattractive in the lower chamber.

### Example 3: Influence of recombinant Slit3 and NETRIN1 on proliferation of OASF and RASF

Proliferation was measured using the Cell Proliferation Kit II (Roche) according to the supplier's instructions. It is based on a colorimetric (XTT) measured non-radioactive quantification of cell proliferation performed for four days. Recombinant Slit3 or NETRIN1 was added in a concentration of 0.1 µg/ml. Cell proliferation assays revealed a calculated doubling time of about 4.5 days (d) for OASF and 3.5 d for RASF. Slit3 treatment resulted in a weak inhibition of proliferation, cell growth decelerated for about 1 d for OASF and RASF after Slit3 treatment (Fig.3). A weak inhibition of cell growth was also seen after NETRIN1 treatment; however, the effect was not significant.

### Example 4: Differentiation of Human Mesenchymal Stem Cells (HMSC)

To evaluate the effect of Slit3 on differentiation HMSC were cultured in pellet culture and treated with Slit3, TGFß or Slit3/TGFß, respectively. Slit3 increased expression of Aggrecan after 7 days of treatment and MIA after 21 days of treatment without induction by TGFß (Fig. 4). TGFß-induced differentiation of HMSCs towards chondrocytes was slightly enhanced by Slit3 treatment analyzing MIA expression (Fig. 4B).

### Example 5: Expression of SLIT3 constructs in an RTS system

To analyse whether also fragments of SLIT3 can be used as a pharmaceutical active ingredient for manufacturing of a pharmaceutical product for treatment of RA various human SLIT3 constructs are generated and tested in functional assays according to Example 1 to 4 using Mel Im cells, A375 cells primary human RASF and OASF cell and HMSC and Example 9. All constructs were made by PCR amplification of cDNA derived from RNA isolated from human chondrocytes or synovia fibroblasts from multiple donors. Construct LRR1-4 (SEQ ID NO: 7) was amplified using the primers 5' GAC CAT ATG GCC CCT GCC CCA CCA AGT GTA CC 3' and 5' GAC CCC GGG ATT GCA TTT GGC CAC AAT G 3', LRR2 (SEQ ID NO: 8) using the primers 5'GAC CAT ATG ATC TCC TGC CCT TCG CCC TGC 3'and 5' GAC CCC GGG GAA GCA CTC GCT GCT GAA CC 3', LRR2 dNC (SEQ ID NO: 9) using the primers 5' GAC CAT ATG ATC GTC GAA ATA CGC CTA GAA C 3'and 5' GAC CCC GGG TGG GTT TTG GGC TAA GTG GAG 3', and LRR3 using the primers 5' GAC CAT ATG GAC CTC GTG TGC CCC GAG AAG 3' and 5' GAC CCC GGG GCT CAG CTG GCA GCT ACT CTC 3'. LRR2 dNCmut (SEQ ID NO: 21), in which the remaining Cys was exchanged by an Ala (Cys85Ala), was cloned by site-directed mutagenesis using the QuickChange Site-Directed Mutagenesis Kit (Stratagene, Amsterdam, Netherlands) and the primers 5' CCA ACA AGA TCA ACG CCC TGC GGG TTA ACA CGT TTC AGG 3' and 5' CCT GAA ACG TGT TAA CCC GCA GGG CGT TGA TCT TGT TGG 3'. Fragments are cloned into the Ndel and Smal site of the expression vector pIVEX2.3-MSC and the insert sequences were verified by sequencing. C-terminal HIS tagged SLIT3 proteins are expressed in an RTS 500 system (Roche Molecular Biochemicals). *In vitro* transcription and translation were performed according to the instruction of the manufacturer. Protein expression was analyzed by Western blot analysis (SDS PAGE) using different amounts (1 µl, 10 µl) of protein generated with the RTS 500 expression system (Fig. 5). The activity of the protein fragments generated with the RTS 500 system was analyzed in a migration assay a) using Mel Im (described by Jacob, et al., 1995), b) A375 (CRL1619, ATCC) cells and c) OASF and RASF cells of Example 2 using a Boyden Chamber assay as described in Example 2 except that 1 µl of protein generated according to the RTS 500 system was added either to the upper or the lower chamber of the system. As depicted in Fig. 6 A and B, Fig. 7 and Fig. 8 there was an inhibition of the migration of all cell lines using Slit3, LRR2, LRR2 dNC and LRR2 dNCmut. Surprisingly, the LRR2 dNC protein which lacks the N- and C-terminal stabilizing part of the SLIT3 protein sequence as well as LRR2 dNCmut (Cys85Ala variant) also inhibits the migration of synovial fibroblast cells indicating that only the minimal binging domain of the protein comprising part of the secondary structure is sufficient for the inhibitory effect.

### Example 6: Animal models for treatment of rheumatoid arthritis (RA)

The migration and destructive behaviour of synovial fibroblasts and the influence of repellent factors of the present invention can be analyzed in different animal models. A well-established model to analyse new biologicals for the treatment of rheumatoid arthritis (RA) is a collagen-induced arthritis (CIA) model in rodents e.g. rat or mice (Bendele et al., 2000). RA is induced in female rats by intradermal/subcutaneous injection of bovine type II collagen in Freund's incomplete adjuvants. As rats develop the disease the repellent factor is administered in a sustained delivery system of e.g. liposomes or saline (PBS) vehicle either subcutaneously or intraperitoneally. Treatment can be analysed by determination of ankle joint swelling and measure of inflammation as well as by histopathological scoring of the joints after decalcification.

An alternative model is the severe-combined immunodeficient mouse (SCID) co-implantation model. Human RASF and fresh human cartilage are-co-implanted together with an inert sponge (e.g. collagen sponge) under the kidney capsule of SCID mice and these are maintained for 60 days (Huber, et al., 2006). The RASF cells invade and destroy the human articular cartilage.

A third model to be used is the antibody-induced arthritis model, where an intraperitoneal injection of a mixture of anti-type II collagen monoclonal antibodies in LPS/PBS induces RA in mice e.g. Balb/c mice (Terato, et al., 1992).

A fourth model is the chronic OVA-induced or antigen induced arthritis model in rabbits (Podolin, et al., 2002). Animals are sensitized by s.c. injection of 10 mg of OVA in 2 ml of CFA and boosted at 3- to 4- week intervals by intrascapula administration of OVA in IFA. Positive skin tested animals are administered OVA in PBS into the knee joint. Rabbits are dosed p.o. with the repellent factor e.g. SLIT3 fragment or with vehicle. To measure the effect of the compound synovial fluids for total cell counts, differential cell analyses, eisosanoid/cytokine measurements and knee diameter using callipers were determined. Blood sample are taken before and after administration of the compound.

In case of testing a compound for its involvement in alteration of synovial fibroblast migration and treatment option for arthritis said compound can be added to the system optionally in saline solution or as a pharmaceutical formulation such as a liposomal preparation.

### Example 7: Animal model for treatment of osteoarthritis (OA)

An animal model for treatment of osteoarthrithis is the rabbit anterior cruciate ligament transection model. Under general anaesthesia and sterile conditions, the knee joint of rabbits are approached via a medial parapatellar incision (between the medial collateral ligament and patellar ligament). The patella is displaced laterally and the infrapatellar fat pad is mobilized and retracted to expose the entire anterior cruciate ligament.

The surgery and injection schedule is as follows: 6 animals per group (sham, vehicle and repellent factor). The animals will be subjected with 1 or multiple (e.g. 5) injections preferably every 10 days. Animals are killed 10 days after the final injection and X-rays are obtained.

Subsequently, samples are prepared for histological examination upon with safranin O-fast green and hematoxylin and eosin staining (e.g. fixed with 10% neutral buffered formaline, decalcification in EDTA and paraffin embedding).

### Example 8: In vivo gene delivery

Gene therapy vectors can be constructed using conventional methods of molecular biology. Non-viral and viral transduction of synovial fibroblasts is described for example in Meinecke et al., 2007 incorporated by reference herewith. Local intra-articular gene therapy can be analyzed in various animal models of rheumatoid arthritis and osteoarthritis such as but not limited to those described under Examples 6 and 7.

Each of the patent applications and patents cited in this text, as well as each document or reference cited in this text are hereby expressly incorporated herein by reference and may be employed in the practice of the invention ("herein cited references"). Each of the cited documents or references as well as patent applications and patents and patents cited within the cited references is hereby expressly incorporated herein by reference.

While preferred embodiments have been illustrated and described, it should be understood that modifications can be made in accordance with ordinary skill in the art without departing from the invention in its broader aspect as defined by the claims.

### Example 9: Inhibition of GAG release

Samples of human cartilage (cube edge length 5mm, 4 cubes each treatment (50mg)) were incubated with or without OA synovial fibroblasts (50.000 cells). Samples with OA SF were additionally treated with either NETRIN1, mSlit3 or LRR2 dNCmut, respectively. Supernatant was taken after 6 days of co-culture and GAG concentration was measured using a commercially available GAG-ELISA (Euro-Diagnostica, Malmo, Sweden; Wieslab sGAG quantitative Kit). Results are shown in Fig. 9.

### Reference List

Bendele AM, Chlipala ES, Scherrer J, Frazier J, Sennello G, Rich WJ and Edwards CK, III (2000) Combination benefit of treatment with the cytokine inhibitors interleukin-1 receptor antagonist and PEGylated soluble tumor necrosis factor receptor type I in animal models of rheumatoid arthritis. Arthritis Rheum 43(12):2648-2659.
Hohenester E, Hussain S and Howitt JA (2006) Interaction of the guidance molecule Slit with cellular receptors. Biochem Soc Trans 34:418-421.
Howitt JA, Clout NJ and Hohenester E (2004) Binding site for Robo receptors revealed by dissection of the leucine-rich repeat region of Slit. Embo J 23:4406-4412.
Huber LC, Distler O, Tamer I, Gay RE, Gay S and Pap T (2006) Synovial fibroblasts: key players in rheumatoid arthritis. Rheumatology (Oxford) 45:669-675.
Itoh A, Miyabayashi T, Ohno M and Sakano S (1998) Cloning and expressions of three mammalian homologues of Drosophila slit suggest possible roles for Slit in the formation and maintenance of the nervous system. Brain Res Mol Brain Res 62:175-186.
Jacob K, Bosserhoff AK, Wach F, Knuchel R, Klein EC, Hein R and Buettner R (1995) Characterization of selected strongly and weakly invasive sublines of a primary human melanoma cell line and isolation of subtractive cDNA clones. Int J Cancer 60(5):668-675.
Katoh Y and Katoh M (2005) Comparative genomics on SLIT1, SLIT2, and SLIT3 orthologs. Oncol Rep 14(5):1351-1355.
Klagsbrun M and Eichmann A (2005) A role for axon guidance receptors and ligands in blood vessel development and tumor angiogenesis. Cytokine Growth Factor Rev 16:535-548.
Kuroda R, Ishida K, Matsumoto T, Akisue T, Fujioka H, Mizuno K, Ohgushi H, Wakitani S and Kurosaka M (2007) Treatment of a full-thickness articular cartilage defect in the femoral condyle of an athlete with autologous bone-marrow stromal cells. Osteoarthritis Cartilage 15(2):226-231.
Little MH, Wilkinson L, Brown DL, Piper M, Yamada T and Stow JL (2001) Dual trafficking of Slit3 to mitochondria and cell surface demonstrates novel localization for Slit protein. Am J Physiol Cell Physiol 281:C486-C495.
Meinecke,I., Rutkauskaite,E., Cinski,A., Muller-Ladner,U., Gay,S., and Pap,T. (2007). Gene transfer to synovial fibroblast: methods and evaluation in the SCID mouse model. Methods Mol. Med. 135: 393-412.
Morlot,C., Hemrika,W., Romijn,R.A., Gros,P., Cusack,S., and McCarthy,A.A. (2007). Production of Slit2 LRR domains in mammalian cells for structural studies and the structure of human Slit2 domain 3. Acta Crystallogr. D. Biol Crystallogr. 63: 961-968.
Pap T (2003) New insights into integrin signalling: implications for rheumatoid arthritis synovial fibroblasts. Arthritis Res Ther 5:154-155.
Pap T (2007) [Regulation of apoptosis in aggressive fibroblasts.]. Z Rheumatol 66:239-242.
Podolin PL, Bolognese BJ, Foley JJ, Schmidt DB, Buckley PT, Widdowson KL, Jin Q, White JR, Lee JM, Goodman RB, Hagen TR, Kajikawa O, Marshall LA, Hay DW and Sarau HM (2002) A potent and selective nonpeptide antagonist of CXCR2 inhibits acute and chronic models of arthritis in the rabbit. J Immunol 169:6435-6444.
Roberts MJ, Bentley MD and Harris JM (2002) Chemistry for peptide and protein PEGylation. Adv Drug Deliv Rev 54:459-476.
Terato K, Hasty KA, Reife RA, Cremer MA, Kang AH and Stuart JM (1992) Induction of arthritis with monoclonal antibodies to collagen. J Immunol 148(7):2103-2108.
Vargesson N, Luria V, Messina I, Erskine L and Laufer E (2001) Expression patterns of Slit and Robo family members during vertebrate limb development. Mech Dev 106:175-180.
Yasuda T (2006) Cartilage destruction by matrix degradation products. Mod Rheumatol 16:197-205.
Zimmermann T, Kunisch E, Pfeiffer R, Hirth A, Stahl HD, Sack U, Laube A, Liesaus E, Roth A, Palombo-Kinne E, Emmrich F and Kinne RW (2001) Isolation and characterization of rheumatoid arthritis synovial fibroblasts from primary culture--primary culture cells markedly differ from fourth-passage cells. Arthritis Res 3:72-76.

## Claims

1. Use of a repellent factor for the manufacture of a pharmaceutical composition for the prevention and/or treatment of a cartilage defect, wherein the repellent factor is selected from Slits.

2. Use according to claim 1, wherein the repellent factor a) has at least 85% homology to the amino acid sequence of SLIT3 (SEQ ID NO:1), b) is an active fragment of SLIT3 or c) has at least 85% homology to the amino acid sequence of the active fragment of b).

3. Use according to any of claims 1 or 2, wherein the repellent factor comprises an amino acid sequence selected from the group consisting of SEQ ID NO:1 to 9 or 21 or has at least 85% homology to any of the amino acid sequences of SEQ ID NO:1 to 9 or 21 and wherein the amino acid sequence is shorter than the mature SLIT3 protein.

4. Use according to any of claims 1 to 3, wherein the repellent factor is a fragment of SLIT3 (SEQ ID NO:1).

5. Use according to claim 1 or 2, wherein the repellent factor is an agonist or antagonist of a ROBO receptor, DCC or UNC receptor.

6. Use according to any of claims 1 to 5, wherein the cartilage defect is associated with infiltration or migration of synovial fibroblasts into articular or non-articular cartilage and/or associated with pannus formation.

7. Use according to any of claims 1 to 6, wherein the cartilage defect is a degenerative disease associated with decreased attachment of cells to articular or non-articular cartilage.

8. Use according to any of claims 1 to 7, wherein the cartilage defect is a degenerative disease, wherein the disease is selected from the group consisting of degenerative disc disease, meniscus tears, anterior crucial ligament (ACL) injury, arthritis, osteoarthritis, rheumatoid arthritis, psoriatic arthritis, juvenile chronic arthritis, rhizomelic pseudoarthritis, rheumatoid polyarthritis, synovitis or villonodular synovitis.

9. Use according to any of claims 1 to 8, wherein the repellent factor is non-glycosylated at least in the second leucine-rich repeat domain or homologous leucine-rich repeat domain.

10. An isolated polypeptide having at least 85% amino acid sequence homology to any of the amino acid sequences of SEQ ID NO:1 to 9 or 21 and wherein the amino acid sequence is shorter than the mature SLIT3 protein for use for the prevention and/or treatment of a cartilage defect.

11. A pharmaceutical composition for use for the prevention and/or treatment of a cartilage defect comprising a repellent factor as defined in any of claims 1 to 9 or a polypeptide of claim 10.

12. Pharmaceutical composition for use according to claim 11 further comprising cartilage cells.

13. Use according to any of claims 1 to 9 or a pharmaceutical composition for use according to any of claims 11 to 12, wherein the pharmaceutical composition is an injectable formulation.

## Patentansprüche

1. Verwendung eines Repellentfaktors zur Herstellung einer pharmazeutischen Zusammensetzung zur Vorbeugung und/oder Behandlung eines Knorpeldefekts, worin der Repellentfaktor aus Slits ausgewählt wird.

2. Verwendung nach Anspruch 1, wobei der Repellentfaktor a) mindestens 85% Homologie zu der Aminosäuresequenz von SLIT3 (SEQ ID NO:1) aufweist, b) ein aktives Fragment von SLIT3 ist oder c) mindestens 85% Homologie zu der Aminosäuresequenz des aktiven Fragments von b) aufweist.

3. Verwendung nach einem der Ansprüche 1 oder 2, worin der Repellentfaktor eine Aminosäuresequenz, ausgewählt aus der Gruppe bestehend aus SEQ ID NO:1 bis 9 oder 21, umfasst oder mindestens 85% Homologie zu einer der Aminosäuresequenzen von SEQ ID NO:1 bis 9 oder 21 aufweist und worin die Aminosäuresequenz kürzer als das mature SLIT3 Protein ist.

4. Verwendung nach einem der Ansprüche 1 bis 3, worin der Repellentfaktor ein Fragment von SLIT3 (SEQ ID NO:1) ist.

5. Verwendung nach Anspruch 1 oder 2, worin der Repellentfaktor ein Agonist oder Antagonist eines ROBO Rezeptors, DCC oder UNC Rezeptors ist.

6. Verwendung nach einem der Ansprüche 1 bis 5, worin der Knorpeldefekt mit Infiltration oder Migration von synovialen Fibroblasten in Gelenks- oder Nichtgelenksknorpel assoziiert ist und/oder mit Pannusbildung assoziiert ist.

7. Verwendung nach einem der Ansprüche 1 bis 6, worin der Knorpeldefekt eine degenerative Erkrankung, welche mit verringerter Anlagerung von Zellen an Gelenks- oder Nichtgelenksknorpel assoziiert ist, ist.

8. Verwendung nach einem der Ansprüche 1 bis 7, worin der Knorpeldefekt eine degenerative Erkrankung ist, worin die Erkrankung von einer Gruppe bestehend aus degenerative Bandscheibenerkrankung, Meniskusrissen, Kreuzband (ACL)-verletzung, Arthritis, Arthrose, rheumatoide Arthritis, Psoriasisarthritis, juvenile chronische Arthritis, rhizomelische Pseudoarthritis, rheumatoide Polyarthritis, Synovitis oder villonoduläre Synovitis ausgewählt wird.

9. Verwendung nach einem der Ansprüche 1 bis 8, worin der Repellentfaktor nichtglykolisiert, zumindest in der zweiten Leucin-reichen Wiederholungsdomäne oder homologen Leucin-reichen Wiederholungsdomäne, ist.

10. Ein isoliertes Polypeptid, welches mindestens 85% Aminosäuresequenzhomologie zu einer der Aminosäuresequenzen von SEQ ID NO:1 bis 9 oder 21 hat und worin die Aminosäuresequenz kürzer als das mature SLIT3 Protein, zur Verwendung zur Vorbeugung und/oder Behandlung von Knorpeldefekten, ist.

11. Eine pharmazeutische Zusammensetzung zur Verwendung zur Vorbeugung und/oder Behandlung eines Knorpeldefekts umfassend einen Repellentfaktor, wie in einem der Ansprüche 1 bis 9 definiert oder eines Polypeptides nach Anspruch 10.

12. Pharmazeutische Zusammensetzung zur Verwendung nach Anspruch 11, weiterhin umfassend Knorpelzellen.

13. Verwendung nach einem der Ansprüche 1 bis 9 oder eine pharmazeutische Zusammensetzung zur Verwendung nach einem der Ansprüche 11 bis 12, worin die pharmazeutische Zusammensetzung eine injizierbare Formulierung ist.

## Revendications

1. Utilisation d'un facteur répulsif pour la fabrication d'une composition pharmaceutique pour la prévention et/ou le traitement d'un défaut du cartilage, dans laquelle le facteur répulsif est sélectionné parmi les slits.

2. Utilisation selon la revendication 1, dans laquelle le facteur répulsif a) possède au moins 85% d'homologie avec la séquence d'acides aminés de SLIT3 (SEQ ID NO: 1), b) est un fragment actif de SLIT3 ou c) possède au moins 85% d'homologie avec la séquence d'acides aminés du fragment actif de b).

3. Utilisation selon l'une quelconque des revendications 1 ou 2, dans laquelle le facteur répulsif comprend une séquence d'acides aminés sélectionnée parmi le groupe constitué de SEQ ID NO:1 à 9 ou 21 ou possède au moins 85% d'homologie avec une quelconque des séquences d'acides aminés de SEQ ID NO:1 à 9 ou 21 et dans laquelle la séquence d'acides aminés est plus courte que la protéine mature SLIT3.

4. Utilisation selon l'une quelconque des revendications 1 à 3, dans laquelle le facteur répulsif est un fragment de SLIT3 (SEQ ID NO:1).

5. Utilisation selon la revendication 1 ou 2, dans laquelle le facteur répulsif est un agoniste ou un antagoniste d'un récepteur ROBO, d'un récepteur DCC ou UNC.

6. Utilisation selon l'une quelconque des revendications 1 à 5, dans laquelle le défaut de cartilage est associé à l'infiltration ou à la migration de fibroblastes synoviaux dans le cartilage articulaire ou non articulaire, et/ou associé à la formation de pannus.

7. Utilisation selon l'une quelconque des revendications 1 à 6, dans laquelle le défaut de cartilage est une maladie dégénérative associée à une diminution de l'attachement des cellules au cartilage articulaire ou non articulaire.

8. Utilisation selon l'une quelconque des revendications 1 à 7, dans laquelle le défaut du cartilage est une maladie dégénérative, dans laquelle la maladie est sélectionnée dans le groupe constitué de la dégénérescence discale, de la déchirure du ménisque, de la lésion des ligaments croisés antérieurs (ACL), de l'arthrite, de l'ostéoarthrite, de l'arthrite rhumatoïde, de l'arthrite psoriasique, de l'arthrite chronique juvénile, de la pseudoarthrite rhizomélique, de la polyarthrite rhumatoïde, de la synovite ou de la synovite villonodulaire.

9. Utilisation selon l'une quelconque des revendications 1 à 8, dans laquelle le facteur répulsif est non glycosylé au moins dans le second domaine de répétition riche en leucine ou le domaine homologue de répétition riche en leucine.

10. Polypeptide isolé ayant au moins 85% d'homologie de séquence d'acides aminés avec les séquences d'acides aminés de SEQ ID NO:1 à 9 ou 21 et dans lequel la séquence d'acides aminés est plus courte que la protéine mature SLIT3 pour l'utilisation pour la prévention et/ou le traitement d'un défaut du cartilage.

11. Composition pharmaceutique pour l'utilisation pour la prévention et/ou le traitement d'un défaut du cartilage comprenant un facteur répulsif comme défini dans l'une quelconque des revendications 1 à 9 ou un polypeptide selon la revendication 10.

12. Composition pharmaceutique pour l'utilisation selon la revendication 11 comprenant en outre des cellules du cartilage.

13. Utilisation selon l'une quelconque des revendications 1 à 9 ou une composition pharmaceutique pour l'utilisation selon l'une quelconque des revendications 11 à 12, dans laquelle la composition pharmaceutique est une formulation injectable.


### SEQUENCE LISTING

<110> Scil Technology GmbH
<120> Neuroendocrine factors for treatment of degenerative diseases
<130> 40794P PCT
<150> 07018131.8
   <151> 2007-09-14
<150> 08006810.9
   <151> 2008-04-03
<160> 21
<170> PatentIn version 3.5
<210> 1
   <211> 1523
   <212> PRT
   <213> Homo sapiens
<400> 1
   Met Ala Pro Gly Trp Ala Gly Val Gly Ala Ala Val Arg Ala Arg Leu
   1               5                   10                  15
   Ala Leu Ala Leu Ala Leu Ala Ser Val Leu Ser Gly Pro Pro Ala Val
               20                  25                  30
   Ala Cys Pro Thr Lys Cys Thr Cys Ser Ala Ala Ser Val Asp Cys His
           35                  40                  45
   Gly Leu Gly Leu Arg Ala Val Pro Arg Gly Ile Pro Arg Asn Ala Glu
       50                  55                  60
   Arg Leu Asp Leu Asp Arg Asn Asn Ile Thr Arg Ile Thr Lys Met Asp
   65                  70                  75                  80
   Phe Ala Gly Leu Lys Asn Leu Arg Val Leu His Leu Glu Asp Asn Gln
                   85                  90                  95
   Val Ser Val Ile Glu Arg Gly Ala Phe Gln Asp Leu Lys Gln Leu Glu
               100                 105                 110
   Arg Leu Arg Leu Asn Lys Asn Lys Leu Gln Val Leu Pro Glu Leu Leu
           115                 120                 125
   Phe Gln Ser Thr Pro Lys Leu Thr Arg Leu Asp Leu Ser Glu Asn Gln
       130                 135                 140
   Ile Gln Gly Ile Pro Arg Lys Ala Phe Arg Gly Ile Thr Asp Val Lys
   145                 150                 155                 160
   Asn Leu Gln Leu Asp Asn Asn His Ile Ser Cys Ile Glu Asp Gly Ala
                   165                 170                 175
   Phe Arg Ala Leu Arg Asp Leu Glu Ile Leu Thr Leu Asn Asn Asn Asn
               180                 185                 190
   Ile Ser Arg Ile Leu Val Thr Ser Phe Asn His Met Pro Lys Ile Arg
           195                 200                 205
   Thr Leu Arg Leu His Ser Asn His Leu Tyr Cys Asp Cys His Leu Ala
       210                 215                 220
   Trp Leu Ser Asp Trp Leu Arg Gln Arg Arg Thr Val Gly Gln Phe Thr
   225                 230                 235                 240
   Leu Cys Met Ala Pro Val His Leu Arg Gly Phe Asn Val Ala Asp Val
                   245                 250                 255
   Gln Lys Lys Glu Tyr Val Cys Pro Ala Pro His Ser Glu Pro Pro Ser
               260                 265                 270
   Cys Asn Ala Asn Ser Ile Ser Cys Pro Ser Pro Cys Thr Cys Ser Asn
           275                 280                 285
   Asn Ile Val Asp Cys Arg Gly Lys Gly Leu Met Glu Ile Pro Ala Asn
       290                 295                 300
   Leu Pro Glu Gly Ile Val Glu Ile Arg Leu Glu Gln Asn Ser Ile Lys
   305                 310                 315                 320
   Ala Ile Pro Ala Gly Ala Phe Thr Gln Tyr Lys Lys Leu Lys Arg Ile
                   325                 330                 335
   Asp Ile Ser Lys Asn Gln Ile Ser Asp Ile Ala Pro Asp Ala Phe Gln
               340                 345                 350
   Gly Leu Lys Ser Leu Thr Ser Leu Val Leu Tyr Gly Asn Lys Ile Thr
           355                 360                 365
   Glu Ile Ala Lys Gly Leu Phe Asp Gly Leu Val Ser Leu Gln Leu Leu
       370                 375                 380
   Leu Leu Asn Ala Asn Lys Ile Asn Cys Leu Arg Val Asn Thr Phe Gln
   385 390 395 400
   Asp Leu Gln Asn Leu Asn Leu Leu Ser Leu Tyr Asp Asn Lys Leu Gln
   405 410 415
   Thr Ile Ser Lys Gly Leu Phe Ala Pro Leu Gln Ser Ile Gln Thr Leu
   420 425 430
   His Leu Ala Gln Asn Pro Phe Val Cys Asp Cys His Leu Lys Trp Leu
   435 440 445
   Ala Asp Tyr Leu Gln Asp Asn Pro Ile Glu Thr Ser Gly Ala Arg Cys
   450 455 460
   Ser Ser Pro Arg Arg Leu Ala Asn Lys Arg Ile Ser Gln Ile Lys Ser
   465 470 475 480
   Lys Lys Phe Arg Cys Ser Gly Ser Glu Asp Tyr Arg Ser Arg Phe Ser
   485 490 495
   Ser Glu Cys Phe Met Asp Leu Val Cys Pro Glu Lys Cys Arg Cys Glu
   500 505 510
   Gly Thr Ile Val Asp Cys Ser Asn Gln Lys Leu Val Arg Ile Pro Ser
   515 520 525
   His Leu Pro Glu Tyr Val Thr Asp Leu Arg Leu Asn Asp Asn Glu Val
   530 535 540
   Ser Val Leu Glu Ala Thr Gly Ile Phe Lys Lys Leu Pro Asn Leu Arg
   545 550 555 560
   Lys Ile Asn Leu Ser Asn Asn Lys Ile Lys Glu Val Arg Glu Gly Ala
   565 570 575
   Phe Asp Gly Ala Ala Ser Val Gln Glu Leu Met Leu Thr Gly Asn Gln
   580 585 590
   Leu Glu Thr Val His Gly Arg Val Phe Arg Gly Leu Ser Gly Leu Lys
   595 600 605
   Thr Leu Met Leu Arg Ser Asn Leu Ile Gly Cys Val Ser Asn Asp Thr
   610 615 620
   Phe Ala Gly Leu Ser Ser Val Arg Leu Leu Ser Leu Tyr Asp Asn Arg
   625 630 635 640
   Ile Thr Thr Ile Thr Pro Gly Ala Phe Thr Thr Leu Val Ser Leu Ser
   645 650 655
   Thr Ile Asn Leu Leu Ser Asn Pro Phe Asn Cys Asn Cys His Leu Ala
   660 665 670
   Trp Leu Gly Lys Trp Leu Arg Lys Arg Arg Ile Val Ser Gly Asn Pro
   675 680 685
   Arg Cys Gln Lys Pro Phe Phe Leu Lys Glu Ile Pro Ile Gln Asp Val
   690 695 700
   Ala Ile Gln Asp Phe Thr Cys Asp Gly Asn Glu Glu Ser Ser Cys Gln
   705 710 715 720
   Leu Ser Pro Arg Cys Pro Glu Gln Cys Thr Cys Met Glu Thr Val Val
   725 730 735
   Arg Cys Ser Asn Lys Gly Leu Arg Ala Leu Pro Arg Gly Met Pro Lys
   740 745 750
   Asp Val Thr Glu Leu Tyr Leu Glu Gly Asn His Leu Thr Ala Val Pro
   755 760 765
   Arg Glu Leu Ser Ala Leu Arg His Leu Thr Leu Ile Asp Leu Ser Asn
   770 775 780
   Asn Ser Ile Ser Met Leu Thr Asn Tyr Thr Phe Ser Asn Met Ser His
   785 790 795 800
   Leu Ser Thr Leu Ile Leu Ser Tyr Asn Arg Leu Arg Cys Ile Pro Val
   805 810 815
   His Ala Phe Asn Gly Leu Arg Ser Leu Arg Val Leu Thr Leu His Gly
   820 825 830
   Asn Asp Ile Ser Ser Val Pro Glu Gly Ser Phe Asn Asp Leu Thr Ser
   835 840 845
   Leu Ser His Leu Ala Leu Gly Thr Asn Pro Leu His Cys Asp Cys Ser
   850 855 860
   Leu Arg Trp Leu Ser Glu Trp Val Lys Ala Gly Tyr Lys Glu Pro Gly
   865 870 875 880
   Ile Ala Arg Cys Ser Ser Pro Glu Pro Met Ala Asp Arg Leu Leu Leu
   885 890 895
   Thr Thr Pro Thr His Arg Phe Gln Cys Lys Gly Pro Val Asp Ile Asn
   900 905 910
   Ile Val Ala Lys Cys Asn Ala Cys Leu Ser Ser Pro Cys Lys Asn Asn
   915 920 925
   Gly Thr Cys Thr Gln Asp Pro Val Glu Leu Tyr Arg Cys Ala Cys Pro
   930 935 940
   Tyr Ser Tyr Lys Gly Lys Asp Cys Thr Val Pro Ile Asn Thr Cys Ile
   945 950 955 960
   Gln Asn Pro Cys Gln His Gly Gly Thr Cys His Leu Ser Asp Ser His
   965 970 975
   Lys Asp Gly Phe Ser Cys Ser Cys Pro Leu Gly Phe Glu Gly Gln Arg
   980 985 990
   Cys Glu Ile Asn Pro Asp Asp Cys Glu Asp Asn Asp Cys Glu Asn Asn
   995 1000 1005
   Ala Thr Cys Val Asp Gly Ile Asn Asn Tyr Val Cys Ile Cys Pro
   1010 1015 1020
   Pro Asn Tyr Thr Gly Glu Leu Cys Asp Glu Val Ile Asp His Cys
   1025 1030 1035
   Val Pro Glu Leu Asn Leu Cys Gln His Glu Ala Lys Cys Ile Pro
   1040 1045 1050
   Leu Asp Lys Gly Phe Ser Cys Glu Cys Val Pro Gly Tyr Ser Gly
   1055 1060 1065
   Lys Leu Cys Glu Thr Asp Asn Asp Asp Cys Val Ala His Lys Cys
   1070 1075 1080
   Arg His Gly Ala Gln Cys Val Asp Thr Ile Asn Gly Tyr Thr Cys
   1085 1090 1095
   Thr Cys Pro Gln Gly Phe Ser Gly Pro Phe Cys Glu His Pro Pro
   1100 1105 1110
   Pro Met Val Leu Leu Gln Thr Ser Pro Cys Asp Gln Tyr Glu Cys
   1115 1120 1125
   Gln Asn Gly Ala Gln Cys Ile Val Val Gln Gln Glu Pro Thr Cys
   1130 1135 1140
   Arg Cys Pro Pro Gly Phe Ala Gly Pro Arg Cys Glu Lys Leu Ile
   1145 1150 1155
   Thr Val Asn Phe Val Gly Lys Asp Ser Tyr Val Glu Leu Ala Ser
   1160 1165 1170
   Ala Lys Val Arg Pro Gln Ala Asn Ile Ser Leu Gln Val Ala Thr
   1175 1180 1185
   Asp Lys Asp Asn Gly Ile Leu Leu Tyr Lys Gly Asp Asn Asp Pro
   1190 1195 1200
   Leu Ala Leu Glu Leu Tyr Gln Gly His Val Arg Leu Val Tyr Asp
   1205 1210 1215
   Ser Leu Ser Ser Pro Pro Thr Thr Val Tyr Ser Val Glu Thr Val
   1220 1225 1230
   Asn Asp Gly Gln Phe His Ser Val Glu Leu Val Thr Leu Asn Gln
   1235 1240 1245
   Thr Leu Asn Leu Val Val Asp Lys Gly Thr Pro Lys Ser Leu Gly
   1250 1255 1260
   Lys Leu Gln Lys Gln Pro Ala Val Gly Ile Asn Ser Pro Leu Tyr
   1265 1270 1275
   Leu Gly Gly Ile Pro Thr Ser Thr Gly Leu Ser Ala Leu Arg Gln
   1280 1285 1290
   Gly Thr Asp Arg Pro Leu Gly Gly Phe His Gly Cys Ile His Glu
   1295 1300 1305
   Val Arg Ile Asn Asn Glu Leu Gln Asp Phe Lys Ala Leu Pro Pro
   1310 1315 1320
   Gln Ser Leu Gly Val Ser Pro Gly Cys Lys Ser Cys Thr Val Cys
   1325 1330 1335
   Lys His Gly Leu Cys Arg Ser Val Glu Lys Asp Ser Val Val Cys
   1340 1345 1350
   Glu Cys Arg Pro Gly Trp Thr Gly Pro Leu Cys Asp Gln Glu Ala
   1355 1360 1365
   Arg Asp Pro Cys Leu Gly His Arg Cys His His Gly Lys Cys Val
   1370 1375 1380
   Ala Thr Gly Thr Ser Tyr Met Cys Lys Cys Ala Glu Gly Tyr Gly
   1385 1390 1395
   Gly Asp Leu Cys Asp Asn Lys Asn Asp Ser Ala Asn Ala Cys Ser
   1400 1405 1410
   Ala Phe Lys Cys His His Gly Gln Cys His Ile Ser Asp Gln Gly
   1415 1420 1425
   Glu Pro Tyr Cys Leu Cys Gln Pro Gly Phe Ser Gly Glu His Cys
   1430 1435 1440
   Gln Gln Glu Asn Pro Cys Leu Gly Gln Val Val Arg Glu Val Ile
   1445 1450 1455
   Arg Arg Gln Lys Gly Tyr Ala Ser Cys Ala Thr Ala Ser Lys Val
   1460 1465 1470
   Pro Ile Met Glu Cys Arg Gly Gly Cys Gly Pro Gln Cys Cys Gln
   1475 1480 1485
   Pro Thr Arg Ser Lys Arg Arg Lys Tyr Val Phe Gln Cys Thr Asp
   1490 1495 1500
   Gly Ser Ser Phe Val Glu Glu Val Glu Arg His Leu Glu Cys Gly
   1505 1510 1515
   Cys Leu Ala Cys Ser
   1520
<210> 2
   <211> 887
   <212> PRT
   <213> Homo sapiens
<400> 2
   Ala Cys Pro Thr Lys Cys Thr Cys Ser Ala Ala Ser Val Asp Cys His
   1               5                   10                  15
   Gly Leu Gly Leu Arg Ala Val Pro Arg Gly Ile Pro Arg Asn Ala Glu
               20                  25                  30
   Arg Leu Asp Leu Asp Arg Asn Asn Ile Thr Arg Ile Thr Lys Met Asp
           35                  40                  45
   Phe Ala Gly Leu Lys Asn Leu Arg Val Leu His Leu Glu Asp Asn Gln
       50                  55                  60
   Val Ser Val Ile Glu Arg Gly Ala Phe Gln Asp Leu Lys Gln Leu Glu
   65                  70                  75                  80
   Arg Leu Arg Leu Asn Lys Asn Lys Leu Gln Val Leu Pro Glu Leu Leu
                   85                  90                  95
   Phe Gln Ser Thr Pro Lys Leu Thr Arg Leu Asp Leu Ser Glu Asn Gln
               100                 105                 110
   Ile Gln Gly Ile Pro Arg Lys Ala Phe Arg Gly Ile Thr Asp Val Lys
           115                 120                 125
   Asn Leu Gln Leu Asp Asn Asn His Ile Ser Cys Ile Glu Asp Gly Ala
       130                 135                 140
   Phe Arg Ala Leu Arg Asp Leu Glu Ile Leu Thr Leu Asn Asn Asn Asn
   145                 150                 155                 160
   Ile Ser Arg Ile Leu Val Thr Ser Phe Asn His Met Pro Lys Ile Arg
                   165                 170                 175
   Thr Leu Arg Leu His Ser Asn His Leu Tyr Cys Asp Cys His Leu Ala
               180                 185                 190
   Trp Leu Ser Asp Trp Leu Arg Gln Arg Arg Thr Val Gly Gln Phe Thr
           195                 200                 205
   Leu Cys Met Ala Pro Val His Leu Arg Gly Phe Asn Val Ala Asp Val
       210                 215                 220
   Gln Lys Lys Glu Tyr Val Cys Pro Ala Pro His Ser Glu Pro Pro Ser
   225 230 235 240
   Cys Asn Ala Asn Ser Ile Ser Cys Pro Ser Pro Cys Thr Cys Ser Asn
   245 250 255
   Asn Ile Val Asp Cys Arg Gly Lys Gly Leu Met Glu Ile Pro Ala Asn
   260 265 270
   Leu Pro Glu Gly Ile Val Glu Ile Arg Leu Glu Gln Asn Ser Ile Lys
   275 280 285
   Ala Ile Pro Ala Gly Ala Phe Thr Gln Tyr Lys Lys Leu Lys Arg Ile
   290 295 300
   Asp Ile Ser Lys Asn Gln Ile Ser Asp Ile Ala Pro Asp Ala Phe Gln
   305 310 315 320
   Gly Leu Lys Ser Leu Thr Ser Leu Val Leu Tyr Gly Asn Lys Ile Thr
   325 330 335
   Glu Ile Ala Lys Gly Leu Phe Asp Gly Leu Val Ser Leu Gln Leu Leu
   340 345 350
   Leu Leu Asn Ala Asn Lys Ile Asn Cys Leu Arg Val Asn Thr Phe Gln
   355 360 365
   Asp Leu Gln Asn Leu Asn Leu Leu Ser Leu Tyr Asp Asn Lys Leu Gln
   370 375 380
   Thr Ile Ser Lys Gly Leu Phe Ala Pro Leu Gln Ser Ile Gln Thr Leu
   385 390 395 400
   His Leu Ala Gln Asn Pro Phe Val Cys Asp Cys His Leu Lys Trp Leu
   405 410 415
   Ala Asp Tyr Leu Gln Asp Asn Pro Ile Glu Thr Ser Gly Ala Arg Cys
   420 425 430
   Ser Ser Pro Arg Arg Leu Ala Asn Lys Arg Ile Ser Gln Ile Lys Ser
   435 440 445
   Lys Lys Phe Arg Cys Ser Gly Ser Glu Asp Tyr Arg Ser Arg Phe Ser
   450 455 460
   Ser Glu Cys Phe Met Asp Leu Val Cys Pro Glu Lys Cys Arg Cys Glu
   465 470 475 480
   Gly Thr Ile Val Asp Cys Ser Asn Gln Lys Leu Val Arg Ile Pro Ser
   485 490 495
   His Leu Pro Glu Tyr Val Thr Asp Leu Arg Leu Asn Asp Asn Glu Val
   500 505 510
   Ser Val Leu Glu Ala Thr Gly Ile Phe Lys Lys Leu Pro Asn Leu Arg
   515 520 525
   Lys Ile Asn Leu Ser Asn Asn Lys Ile Lys Glu Val Arg Glu Gly Ala
   530 535 540
   Phe Asp Gly Ala Ala Ser Val Gln Glu Leu Met Leu Thr Gly Asn Gln
   545 550 555 560
   Leu Glu Thr Val His Gly Arg Val Phe Arg Gly Leu Ser Gly Leu Lys
   565 570 575
   Thr Leu Met Leu Arg Ser Asn Leu Ile Gly Cys Val Ser Asn Asp Thr
   580 585 590
   Phe Ala Gly Leu Ser Ser Val Arg Leu Leu Ser Leu Tyr Asp Asn Arg
   595 600 605
   Ile Thr Thr Ile Thr Pro Gly Ala Phe Thr Thr Leu Val Ser Leu Ser
   610 615 620
   Thr Ile Asn Leu Leu Ser Asn Pro Phe Asn Cys Asn Cys His Leu Ala
   625 630 635 640
   Trp Leu Gly Lys Trp Leu Arg Lys Arg Arg Ile Val Ser Gly Asn Pro
   645 650 655
   Arg Cys Gln Lys Pro Phe Phe Leu Lys Glu Ile Pro Ile Gln Asp Val
   660 665 670
   Ala Ile Gln Asp Phe Thr Cys Asp Gly Asn Glu Glu Ser Ser Cys Gln
   675 680 685
   Leu Ser Pro Arg Cys Pro Glu Gln Cys Thr Cys Met Glu Thr Val Val
   690 695 700
   Arg Cys Ser Asn Lys Gly Leu Arg Ala Leu Pro Arg Gly Met Pro Lys
   705 710 715 720
   Asp Val Thr Glu Leu Tyr Leu Glu Gly Asn His Leu Thr Ala Val Pro
   725 730 735
   Arg Glu Leu Ser Ala Leu Arg His Leu Thr Leu Ile Asp Leu Ser Asn
   740 745 750
   Asn Ser Ile Ser Met Leu Thr Asn Tyr Thr Phe Ser Asn Met Ser His
   755 760 765
   Leu Ser Thr Leu Ile Leu Ser Tyr Asn Arg Leu Arg Cys Ile Pro Val
   770 775 780
   His Ala Phe Asn Gly Leu Arg Ser Leu Arg Val Leu Thr Leu His Gly
   785 790 795 800
   Asn Asp Ile Ser Ser Val Pro Glu Gly Ser Phe Asn Asp Leu Thr Ser
   805 810 815
   Leu Ser His Leu Ala Leu Gly Thr Asn Pro Leu His Cys Asp Cys Ser
   820 825 830
   Leu Arg Trp Leu Ser Glu Trp Val Lys Ala Gly Tyr Lys Glu Pro Gly
   835 840 845
   Ile Ala Arg Cys Ser Ser Pro Glu Pro Met Ala Asp Arg Leu Leu Leu
   850 855 860
   Thr Thr Pro Thr His Arg Phe Gln Cys Lys Gly Pro Val Asp Ile Asn
   865 870 875 880
   Ile Val Ala Lys Cys Asn Ala
   885
<210> 3
   <211> 244
   <212> PRT
   <213> Homo sapiens
<400> 3
   Ala Cys Pro Thr Lys Cys Thr Cys Ser Ala Ala Ser Val Asp Cys His
   1 5 10 15
   Gly Leu Gly Leu Arg Ala Val Pro Arg Gly Ile Pro Arg Asn Ala Glu
   20 25 30
   Arg Leu Asp Leu Asp Arg Asn Asn Ile Thr Arg Ile Thr Lys Met Asp
   35 40 45
   Phe Ala Gly Leu Lys Asn Leu Arg Val Leu His Leu Glu Asp Asn Gln
   50 55 60
   Val Ser Val Ile Glu Arg Gly Ala Phe Gln Asp Leu Lys Gln Leu Glu
   65 70 75 80
   Arg Leu Arg Leu Asn Lys Asn Lys Leu Gln Val Leu Pro Glu Leu Leu
   85 90 95
   Phe Gln Ser Thr Pro Lys Leu Thr Arg Leu Asp Leu Ser Glu Asn Gln
   100 105 110
   Ile Gln Gly Ile Pro Arg Lys Ala Phe Arg Gly Ile Thr Asp Val Lys
   115 120 125
   Asn Leu Gln Leu Asp Asn Asn His Ile Ser Cys Ile Glu Asp Gly Ala
   130 135 140
   Phe Arg Ala Leu Arg Asp Leu Glu Ile Leu Thr Leu Asn Asn Asn Asn
   145 150 155 160
   Ile Ser Arg Ile Leu Val Thr Ser Phe Asn His Met Pro Lys Ile Arg
   165 170 175
   Thr Leu Arg Leu His Ser Asn His Leu Tyr Cys Asp Cys His Leu Ala
   180 185 190
   Trp Leu Ser Asp Trp Leu Arg Gln Arg Arg Thr Val Gly Gln Phe Thr
   195 200 205
   Leu Cys Met Ala Pro Val His Leu Arg Gly Phe Asn Val Ala Asp Val
   210 215 220
   Gln Lys Lys Glu Tyr Val Cys Pro Ala Pro His Ser Glu Pro Pro Ser
   225 230 235 240
   Cys Asn Ala Asn
<210> 4
   <211> 221
   <212> PRT
   <213> Homo sapiens
<400> 4
   Asp Leu Val Cys Pro Glu Lys Cys Arg Cys Glu Gly Thr Ile Val Asp
   1               5                   10                  15
   Cys Ser Asn Gln Lys Leu Val Arg Ile Pro Ser His Leu Pro Glu Tyr
               20                  25                  30
   Val Thr Asp Leu Arg Leu Asn Asp Asn Glu Val Ser Val Leu Glu Ala
           35                  40                  45
   Thr Gly Ile Phe Lys Lys Leu Pro Asn Leu Arg Lys Ile Asn Leu Ser
       50                  55                  60
   Asn Asn Lys Ile Lys Glu Val Arg Glu Gly Ala Phe Asp Gly Ala Ala
   65                  70                  75                  80
   Ser Val Gln Glu Leu Met Leu Thr Gly Asn Gln Leu Glu Thr Val His
                   85                  90                  95
   Gly Arg Val Phe Arg Gly Leu Ser Gly Leu Lys Thr Leu Met Leu Arg
               100                 105                 110
   Ser Asn Leu Ile Gly Cys Val Ser Asn Asp Thr Phe Ala Gly Leu Ser
           115                 120                 125
   Ser Val Arg Leu Leu Ser Leu Tyr Asp Asn Arg Ile Thr Thr Ile Thr
       130                 135                 140
   Pro Gly Ala Phe Thr Thr Leu Val Ser Leu Ser Thr Ile Asn Leu Leu
   145                 150                 155                 160
   Ser Asn Pro Phe Asn Cys Asn Cys His Leu Ala Trp Leu Gly Lys Trp
                   165                 170                 175
   Leu Arg Lys Arg Arg Ile Val Ser Gly Asn Pro Arg Cys Gln Lys Pro
               180                 185                 190
   Phe Phe Leu Lys Glu Ile Pro Ile Gln Asp Val Ala Ile Gln Asp Phe
           195                 200                 205
   Thr Cys Asp Gly Asn Glu Glu Ser Ser Cys Gln Leu Ser
       210                 215                 220
<210> 5
   <211> 196
   <212> PRT
   <213> Homo sapiens
<400> 5
   Pro Arg Cys Pro Glu Gln Cys Thr Cys Met Glu Thr Val Val Arg Cys
   1               5                   10                  15
   Ser Asn Lys Gly Leu Arg Ala Leu Pro Arg Gly Met Pro Lys Asp Val
               20                  25                  30
   Thr Glu Leu Tyr Leu Glu Gly Asn His Leu Thr Ala Val Pro Arg Glu
           35                  40                  45
   Leu Ser Ala Leu Arg His Leu Thr Leu Ile Asp Leu Ser Asn Asn Ser
       50                  55                  60
   Ile Ser Met Leu Thr Asn Tyr Thr Phe Ser Asn Met Ser His Leu Ser
   65                  70                  75                  80
   Thr Leu Ile Leu Ser Tyr Asn Arg Leu Arg Cys Ile Pro Val His Ala
                   85                  90                  95
   Phe Asn Gly Leu Arg Ser Leu Arg Val Leu Thr Leu His Gly Asn Asp
               100                 105                 110
   Ile Ser Ser Val Pro Glu Gly Ser Phe Asn Asp Leu Thr Ser Leu Ser
           115                 120                 125
   His Leu Ala Leu Gly Thr Asn Pro Leu His Cys Asp Cys Ser Leu Arg
       130                 135                 140
   Trp Leu Ser Glu Trp Val Lys Ala Gly Tyr Lys Glu Pro Gly Ile Ala
   145                 150                 155                 160
   Arg Cys Ser Ser Pro Glu Pro Met Ala Asp Arg Leu Leu Leu Thr Thr
                   165                 170                 175
   Pro Thr His Arg Phe Gln Cys Lys Gly Pro Val Asp Ile Asn Ile Val
               180                 185                 190
   Ala Lys Cys Asn
           195
<210> 6
   <211> 225
   <212> PRT
   <213> Homo sapiens
<400> 6
   Ser Ile Ser Cys Pro Ser Pro Cys Thr Cys Ser Asn Asn Ile Val Asp
   1               5                   10                  15
   Cys Arg Gly Lys Gly Leu Met Glu Ile Pro Ala Asn Leu Pro Glu Gly
               20                  25                  30
   Ile Val Glu Ile Arg Leu Glu Gln Asn Ser Ile Lys Ala Ile Pro Ala
           35                  40                  45
   Gly Ala Phe Thr Gln Tyr Lys Lys Leu Lys Arg Ile Asp Ile Ser Lys
       50                  55                  60
   Asn Gln Ile Ser Asp Ile Ala Pro Asp Ala Phe Gln Gly Leu Lys Ser
   65                  70                  75                  80
   Leu Thr Ser Leu Val Leu Tyr Gly Asn Lys Ile Thr Glu Ile Ala Lys
                   85                  90                  95
   Gly Leu Phe Asp Gly Leu Val Ser Leu Gln Leu Leu Leu Leu Asn Ala
               100                 105                 110
   Asn Lys Ile Asn Cys Leu Arg Val Asn Thr Phe Gln Asp Leu Gln Asn
           115                 120                 125
   Leu Asn Leu Leu Ser Leu Tyr Asp Asn Lys Leu Gln Thr Ile Ser Lys
       130                 135                 140
   Gly Leu Phe Ala Pro Leu Gln Ser Ile Gln Thr Leu His Leu Ala Gln
   145                 150                 155                 160
   Asn Pro Phe Val Cys Asp Cys His Leu Lys Trp Leu Ala Asp Tyr Leu
                   165                 170                 175
   Gln Asp Asn Pro Ile Glu Thr Ser Gly Ala Arg Cys Ser Ser Pro Arg
               180                 185                 190
   Arg Leu Ala Asn Lys Arg Ile Ser Gln Ile Lys Ser Lys Lys Phe Arg
           195                 200                 205
   Cys Ser Gly Ser Glu Asp Tyr Arg Ser Arg Phe Ser Ser Glu Cys Phe
   210 215 220
   Met
   225
<210> 8
   <211> 223
   <212> PRT
   <213> Homo sapiens
<400> 8
   Ile Ser Cys Pro Ser Pro Cys Thr Cys Ser Asn Asn Ile Val Asp Cys
   1               5                   10                  15
   Arg Gly Lys Gly Leu Met Glu Ile Pro Ala Asn Leu Pro Glu Gly Ile
               20                  25                  30
   Val Glu Ile Arg Leu Glu Gln Asn Ser Ile Lys Ala Ile Pro Ala Gly
           35                  40                  45
   Ala Phe Thr Gln Tyr Lys Lys Leu Lys Arg Ile Asp Ile Ser Lys Asn
       50                  55                  60
   Gln Ile Ser Asp Ile Ala Pro Asp Ala Phe Gln Gly Leu Lys Ser Leu
   65                  70                  75                  80
   Thr Ser Leu Val Leu Tyr Gly Asn Lys Ile Thr Glu Ile Ala Lys Gly
                   85                  90                  95
   Leu Phe Asp Gly Leu Val Ser Leu Gln Leu Leu Leu Leu Asn Ala Asn
               100                 105                 110
   Lys Ile Asn Cys Leu Arg Val Asn Thr Phe Gln Asp Leu Gln Asn Leu
           115                 120                 125
   Asn Leu Leu Ser Leu Tyr Asp Asn Lys Leu Gln Thr Ile Ser Lys Gly
       130                 135                 140
   Leu Phe Ala Pro Leu Gln Ser Ile Gln Thr Leu His Leu Ala Gln Asn
   145                 150                 155                 160
   Pro Phe Val Cys Asp Cys His Leu Lys Trp Leu Ala Asp Tyr Leu Gln
                   165                 170                 175
   Asp Asn Pro Ile Glu Thr Ser Gly Ala Arg Cys Ser Ser Pro Arg Arg
               180                 185                 190
   Leu Ala Asn Lys Arg Ile Ser Gln Ile Lys Ser Lys Lys Phe Arg Cys
           195                 200                 205
   Ser Gly Ser Glu Asp Tyr Arg Ser Arg Phe Ser Ser Glu Cys Phe
       210                 215                 220
<210> 9
   <211> 130
   <212> PRT
   <213> Homo sapiens
<400> 9
   Ile Val Glu Ile Arg Leu Glu Gln Asn Ser Ile Lys Ala Ile Pro Ala
   1               5                   10                  15
   Gly Ala Phe Thr Gln Tyr Lys Lys Leu Lys Arg Ile Asp Ile Ser Lys
               20                  25                  30
   Asn Gln Ile Ser Asp Ile Ala Pro Asp Ala Phe Gln Gly Leu Lys Ser
           35                  40                  45
   Leu Thr Ser Leu Val Leu Tyr Gly Asn Lys Ile Thr Glu Ile Ala Lys
       50                  55                  60
   Gly Leu Phe Asp Gly Leu Val Ser Leu Gln Leu Leu Leu Leu Asn Ala
   65                  70                  75                  80
   Asn Lys Ile Asn Cys Leu Arg Val Asn Thr Phe Gln Asp Leu Gln Asn
                   85                  90                  95
   Leu Asn Leu Leu Ser Leu Tyr Asp Asn Lys Leu Gln Thr Ile Ser Lys
               100                 105                 110
   Gly Leu Phe Ala Pro Leu Gln Ser Ile Gln Thr Leu His Leu Ala Gln
           115                 120                 125
   Asn Pro
       130
<210> 10
   <211> 603
   <212> PRT
   <213> Homo sapiens
<400> 10
   Met Met Arg Ala Val Trp Glu Ala Leu Ala Ala Leu Ala Ala Val Ala
   1               5                   10                  15
   Cys Leu Val Gly Ala Val Arg Gly Gly Pro Gly Leu Ser Met Phe Ala
               20                  25                  30
   Gly Gln Ala Ala Gln Pro Asp Pro Cys Ser Asp Glu Asn Gly His Pro
   35 40 45
   Arg Arg Cys Ile Pro Asp Phe Val Asn Ala Ala Phe Gly Lys Asp Val
   50 55 60
   Arg Val Ser Ser Thr Cys Gly Arg Pro Pro Ala Arg Tyr Cys Val Val
   65 70 75 80
   Ser Glu Arg Gly Glu Glu Arg Leu Arg Ser Cys His Leu Cys Asn Ala
   85 90 95
   Ser Asp Pro Lys Lys Ala His Pro Pro Ala Phe Leu Thr Asp Leu Asn
   100 105 110
   Asn Pro His Asn Leu Thr Cys Trp Gln Ser Glu Asn Tyr Leu Gln Phe
   115 120 125
   Pro His Asn Val Thr Leu Thr Leu Ser Leu Gly Lys Lys Phe Glu Val
   130 135 140
   Thr Tyr Val Ser Leu Gln Phe Cys Ser Pro Arg Pro Glu Ser Met Ala
   145 150 155 160
   Ile Tyr Lys Ser Met Asp Tyr Gly Arg Thr Trp Val Pro Phe Gln Phe
   165 170 175
   Tyr Ser Thr Gln Cys Arg Lys Met Tyr Asn Arg Pro His Arg Ala Pro
   180 185 190
   Ile Thr Lys Gln Asn Glu Gln Glu Ala Val Cys Thr Asp Ser His Thr
   195 200 205
   Asp Met Arg Pro Leu Ser Gly Gly Leu Ile Ala Phe Ser Thr Leu Asp
   210 215 220
   Gly Arg Pro Ser Ala His Asp Phe Asp Asn Ser Pro Val Leu Gln Asp
   225 230 235 240
   Trp Val Thr Ala Thr Asp Ile Arg Val Ala Phe Ser Arg Leu His Thr
   245 250 255
   Phe Gly Asp Glu Asn Glu Asp Asp Ser Glu Leu Ala Arg Asp Ser Tyr
   260 265 270
   Phe Tyr Ala Val Ser Asp Leu Gln Val Gly Gly Arg Cys Lys Cys Asn
           275                 280                 285
   Gly His Ala Ala Arg Cys Val Arg Asp Arg Asp Asp Ser Leu Val Cys
       290                 295                 300
   Asp Cys Arg His Asn Thr Ala Gly Pro Glu Cys Asp Arg Cys Lys Pro
   305                 310                 315                 320
   Phe His Tyr Asp Arg Pro Trp Gln Arg Ala Thr Ala Arg Glu Ala Asn
                   325                 330                 335
   Glu Cys Val Ala Cys Asn Cys Asn Leu His Ala Arg Arg Cys Arg Phe
               340                 345                 350
   Asn Met Glu Leu Tyr Lys Leu Ser Gly Arg Lys Ser Gly Gly Val Cys
           355                 360                 365
   Leu Asn Cys Arg His Asn Thr Ala Gly Arg His Cys His Tyr Cys Lys
       370                 375                 380
   Glu Gly Tyr Tyr Arg Asp Gly Lys Pro Ile Thr His Arg Lys Ala Cys
   385                 390                 395                 400
   Lys Ala Cys Asp Cys His Pro Val Gly Ala Ala Gly Lys Thr Cys Asn
                   405                 410                 415
   Gln Thr Thr Gly Gln Cys Pro Cys Lys Asp Gly Val Thr Gly Ile Thr
               420                 425                 430
   Cys Asn Arg Cys Ala Lys Gly Tyr Gln Gln Ser Arg Ser Pro Ile Ala
           435                 440                 445
   Pro Cys Ile Lys Ile Pro Val Ala Pro Pro Thr Thr Ala Ala Ser Ser
       450                 455                 460
   Val Glu Glu Pro Glu Asp Cys Asp Ser Tyr Cys Lys Ala Ser Lys Gly
   465                 470                 475                 480
   Lys Leu Lys Ile Asn Met Lys Lys Tyr Cys Lys Lys Asp Tyr Ala Val
                   485                 490                 495
   Gln Ile His Ile Leu Lys Ala Asp Lys Ala Gly Asp Trp Trp Lys Phe
               500                 505                 510
   Thr Val Asn Ile Ile Ser Val Tyr Lys Gln Gly Thr Ser Arg Ile Arg
   515 520 525
   Arg Gly Asp Gln Ser Leu Trp Ile Arg Ser Arg Asp Ile Ala Cys Lys
   530 535 540
   Cys Pro Lys Ile Lys Pro Leu Lys Lys Tyr Leu Leu Leu Gly Asn Ala
   545 550 555 560
   Glu Asp Ser Pro Asp Gln Ser Gly Ile Val Ala Asp Lys Ser Ser Leu
   565 570 575
   Val Ile Gln Trp Arg Asp Thr Trp Ala Arg Arg Leu Arg Lys Phe Gln
   580 585 590
   Gln Arg Glu Lys Lys Gly Lys Cys Lys Lys Ala
   595 600
<210> 11
   <211> 32
   <212> DNA
   <213> Homo sapiens
<220>
   <221> misc_feature
   <222> (1)..(32)
<400> 11
   gaccatatgg cccctgcccc accaagtgta cc
   32
<210> 12
   <211> 28
   <212> DNA
   <213> Homo sapiens
<220>
   <221> misc_feature
   <222> (1)..(28)
<400> 12
   gaccccggga ttgcatttgg ccacaatg
   28
<210> 13
   <211> 30
   <212> DNA
   <213> Homo sapiens
<220>
   <221> misc_feature
   <222> (1)..(30)
<400> 13
   gaccatatga tctcctgccc ttcgccctgc
   30
<210> 14
   <211> 29
   <212> DNA
   <213> Homo sapiens
<220>
   <221> misc_feature
   <222> (1)..(29)
<400> 14
   gaccccgggg aagcactcgc tgctgaacc
   29
<210> 15
   <211> 31
   <212> DNA
   <213> Homo sapiens
<220>
   <221> misc_feature
   <222> (1)..(31)
<400> 15
   gaccatatga tcgtcgaaat acgcctagaa c
   31
<210> 16
   <211> 30
   <212> DNA
   <213> Homo sapiens
<220>
   <221> misc_feature
   <222> (1)..(30)
<400> 16
   gaccccgggt gggttttggg ctaagtggag
   30
<210> 17
   <211> 30
   <212> DNA
   <213> Homo sapiens
<220>
   <221> misc_feature
   <222> (1)..(30)
<400> 17
   gaccatatgg acctcgtgtg ccccgagaag
   30
<210> 18
   <211> 30
   <212> DNA
   <213> Homo sapiens
<220>
   <221> misc_feature
   <222> (1)..(30)
<400> 18
   gaccccgggg ctcagctggc agctactctc
   30
<210> 19
   <211> 39
   <212> DNA
   <213> Homo sapiens
<220>
   <221> misc_feature
   <222> (1)..(39)
<400> 19
   ccaacaagat caacgccctg cgggttaaca cgtttcagg 39
<210> 20
   <211> 39
   <212> DNA
   <213> Homo sapiens
<220>
   <221> misc_feature
   <222> (1)..(39)
<400> 20
   cctgaaacgt gttaacccgc agggcgttga tcttgttgg
   39
<210> 21
   <211> 130
   <212> PRT
   <213> Homo sapiens
<400> 21
   Ile Val Glu Ile Arg Leu Glu Gln Asn Ser Ile Lys Ala Ile Pro Ala
   1               5                   10                  15
   Gly Ala Phe Thr Gln Tyr Lys Lys Leu Lys Arg Ile Asp Ile Ser Lys
               20                  25                  30
   Asn Gln Ile Ser Asp Ile Ala Pro Asp Ala Phe Gln Gly Leu Lys Ser
           35                  40                  45
   Leu Thr Ser Leu Val Leu Tyr Gly Asn Lys Ile Thr Glu Ile Ala Lys
       50                  55                  60
   Gly Leu Phe Asp Gly Leu Val Ser Leu Gln Leu Leu Leu Leu Asn Ala
   65                  70                  75                  80
   Asn Lys Ile Asn Ala Leu Arg Val Asn Thr Phe Gln Asp Leu Gln Asn
                   85                  90                  95
   Leu Asn Leu Leu Ser Leu Tyr Asp Asn Lys Leu Gln Thr Ile Ser Lys
               100                 105                 110
   Gly Leu Phe Ala Pro Leu Gln Ser Ile Gln Thr Leu His Leu Ala Gln
           115                 120                 125
   Asn Pro
       130